# EUROPEAN PATENT APPLICATION

(11) **EP 4 793 280 A1**
(43) Date of publication of application: **19.08.2026**
(21) Application number: 24876723.8
(22) Date of filing: 14.10.2024
(51) Int. Cl.: C07K 7/04, C07K 19/00, C07K 14/33, C12N 15/62

(54) **RECOMBINANT POLYPEPTIDE AND METHOD FOR PREPARING NEUROTOXIN**

(30) Priority: 13.10.2023 CN 202311328450
(71) Applicant: Beijing Botuvac Biotechnology Co., Ltd., Beijing 102629 (CN)
(72) Inventor: WU, Yongqiang, Beijing 102629 (CN); SHI, Wen, Beijing 102629 (CN); LI, Jia, Beijing 102629 (CN); ZHANG, Huajie, Beijing 102629 (CN); WEI, Chen, Beijing 102629 (CN); CHEN, Guangming, Beijing 102629 (CN); ZHANG, Chenghong, Beijing 102629 (CN)
(74) Representative: Icosa
(86) International application number: PCT/CN2024/124685
(87) International publication number: WO 2025/077918

(57) **Abstract**

A recombinant polypeptide and a method for using the recombinant polypeptide to produce a neurotoxin.

## Description

### CROSS REFERENCE TO RELATED APPLICATIONS

This application claims the priority of Chinese Patent Application No. 202311328450.9, filed with the China National Intellectual Property Administration on October 13, 2023, and titled with "POLYPEPTIDES FOR EFFICIENT AND RAPID RECOMBINANT EXPRESSION OF NEUROTOXIN PROTEINS AND METHODS THEREOF", which is hereby incorporated by reference in its entirety.

### FIELD

The present disclosure relates to the field of neurotoxin, especially relates to a recombinant polypeptide comprising a light chain of neurotoxin and/or a heavy chain of neurotoxin, and a linker peptide, and a method for producing a neurotoxin by recombinant expression of the polypeptide.

### BACKGROUND

Neurotoxins are chemical substances that primarily act on ion channels. Such toxins can act at the junction between motor neurons and muscles, thereby preventing contraction of striated muscles, which in severe cases may result in respiratory paralysis and death by asphyxiation. Certain organisms in nature contain or are capable of releasing such toxins, for example botulinum neurotoxin (BoNT), tetanus neurotoxin (TeNT), and diphtheria toxin.

Botulinum neurotoxins (BoNTs), also referred to as botulinum toxins (BT), are neurotoxic proteins produced by the Gram-positive *Clostridium botulinum* during its growth. Its toxicity is 10,000 times that of potassium cyanide, and the minimum lethal dose for humans is about 0.1 µg. Based on differences in toxicity and protein antigenicity, botulinum neurotoxins are classified into seven major classical serotypes, namely serotypes A, B, C, D, E, F, and G (BoNT/A to BoNT/G). Among them, botulinum toxin serotype A (also referred to as botulinum toxin A), produced by *Clostridium botulinum* type A, exhibits the highest toxicity, whereas serotypes C and D mainly cause poisoning in livestock, poultry, and wild birds.

The structures of the various serotypes of BoNT are substantially identical, each of which is a double-chain structure consisting of two subunits, a light chain (LC) and a heavy chain (HC), linked by a disulfide bond, including an N-terminal light chain (LC) and a C-terminal heavy chain (HC). The LC is a zinc metalloprotease with a relative molecular mass of 50 kDa and is the active component of BoNT; the HC has a relative molecular mass of 100 kDa and consists of an N-terminal translocation domain (HN) and a C-terminal cell-binding domain (HC), serving as the transport vehicle for BoNT. Other neurotoxins, such as tetanus neurotoxin and diphtheria toxin, also possess a double-chain structure consisting of two subunits, a light chain and a heavy chain, linked via a disulfide bond.

BoNT is well known for its ability to induce muscle relaxation and paralysis. In particular, BoNT/A has been applied in various medical and cosmetic procedures, including the treatment of glabellar lines or hyperkinetic facial wrinkles, migraine, hemifacial spasm, overactive bladder, hyperhidrosis, nasolabial folds, cervical dystonia, and facial spasm and rigidity. Due to the potent neurotoxicity of BoNT, with a median lethal dose (LD₅₀) of 1 ng/kg, the production and marketing of botulinum toxin products are strictly regulated in various countries.

As of 2022, only botulinum toxin serotypes A and B have been developed for commercial use. Among them, serotype A exhibits the highest potency, and approximately 20 botulinum toxin serotype A products have been approved for marketing worldwide (Journal of Neural Transmission (2022) 129:829-833). Serotype B is mainly used in patients who have developed antibodies against botulinum toxin serotype A, and globally only one botulinum toxin serotype B product, MyoBloc produced by Solstice, has been approved by the U.S. Food and Drug Administration (FDA) (J Neurol 2003, 249:1729-1732).

At present, all marketed botulinum toxin products are derived from *Clostridium botulinum*. Due to limitations associated with the high toxicity of the *Clostridium botulinum*, expression levels and purification processes, the production of such products has long manufacturing cycles, high costs, and relatively poor safety during the production process.

Therefore, there remains a need to develop a recombinant botulinum toxin and a production method thereof, characterized by high yield and purity, improved safety due to the absence of additional amino acids, universal applicability, and simplified processes.

### SUMMARY

The inventors unexpectedly find that a complete neurotoxin can be obtained by cleaving a recombinant polypeptide used for the production of the neurotoxin using a protease, which recognizes and cleaves a linker peptide in the recombinant polypeptide, without causing significant erroneous cleavage at protease cleavage sites within the light and heavy chains of the neurotoxin. The present disclosure is completed on this basis.

An objective of the present disclosure is to provide a recombinant polypeptide for producing neurotoxins, production method, and use thereof to address the above problems in the existing neurotoxin production process.

In the first aspect, the present disclosure provides a recombinant polypeptide, comprising a light chain of a neurotoxin and/or a heavy chain of a neurotoxin, and a linker peptide;
wherein the linker peptide comprises a tag peptide and at least one or two protease cleavage site sequences selected from the group consisting of: a first protease cleavage site sequence and a second protease cleavage site sequence;
wherein, after the first protease cleavage site sequence is recognized by a protease, the protease cleaves the recombinant polypeptide at a junction between the light chain and the linker peptide, and/or after the second protease cleavage site sequence is recognized by a protease, the protease cleaves the recombinant polypeptide at a junction between the linker peptide and the heavy chain.

In one embodiment, the recombinant polypeptide comprises a light chain of a neurotoxin, a heavy chain of a neurotoxin and a linker peptide located between the light chain and the heavy chain, for example, the recombinant polypeptide comprises, from N-terminus to C-terminus, the light chain of the neurotoxin, the linker peptide and the heavy chain of the neurotoxin, wherein the light chain and the heavy chain are linked into a single polypeptide chain by the linker peptide.

In another embodiment, the recombinant polypeptide comprises a light chain of a neurotoxin linked to a linker peptide, for example, the recombinant polypeptide comprises, from N-terminus to C-terminus, the light chain of the neurotoxin and the linker peptide, and/or
the recombinant polypeptide comprises a heavy chain of a neurotoxin linked to a linker peptide, for example, the recombinant polypeptide comprises, from N-terminus to C-terminus, the linker peptide and the heavy chain of the neurotoxin,
wherein the linker peptide linked to the light chain and the linker peptide linked to the heavy chain are identical or different.

In a specific embodiment, the light chain and the heavy chain are located in separate polypeptide chains.

In another embodiment, the neurotoxin is selected from the group consisting of botulinum neurotoxin, tetanus neurotoxin, and diphtheria toxin. For example, the botulinum neurotoxin is selected from the group consisting of botulinum neurotoxin serotypes A, B, C, D, E, F and G, preferably selected from the group consisting of botulinum neurotoxin serotypes A, B and E, for example botulinum neurotoxin serotype A.

In a specific embodiment, the light chain has an amino acid sequence as set forth in SEQ ID NO: 1, and/or the heavy chain has an amino acid sequence as set forth in SEQ ID NO: 2. The light chain and/or the heavy chain may further are sequence variants having at least 60%, 70%, 80%, 90%, 95%, 96%, 97%, 98%, or 99% identity to the sequence as set forth in SEQ ID NO: 1 or SEQ ID NO: 2, respectively, and retaining botulinum toxin activity. Such sequence variants may be naturally occurring variants or artificially modified variants.

In a specific embodiment, the first protease cleavage site sequence and the second protease cleavage site sequence are each independently selected from the group consisting of cleavage site sequences of Kex2 protease, EK protease and TEV protease; for example, wherein the first protease cleavage site sequence is selected from the group consisting of R, K, RR, KK, RK and KR, and/or the second protease cleavage site sequence is selected from the group consisting of R, K, RR, KK, RK, KR and DDDDK.

In a specific embodiment, both the first protease cleavage site sequence and the second protease cleavage site sequence are a Kex2 protease cleavage site sequence.

In another embodiment, the tag peptide is selected from the group consisting of a polyhistidine tag (poly(His)-tag), a FLAG tag, a streptavidin-binding tag (Strep-tag) and a glutathione S-transferase tag (GST-tag). In a specific embodiment, the tag peptide is a polyhistidine tag (poly(His)-tag), for example, a 4, 5, 6, 7, 8, 9 or 10×His-tag .

In another embodiment, the recombinant polypeptide further comprises a first auxiliary sequence, wherein the first auxiliary sequence is located between the first protease cleavage site sequence and the tag peptide. Furthermore, the second auxiliary sequence is located between the second protease cleavage site sequence and the tag peptide.

In a specific embodiment, the first auxiliary sequence and the second auxiliary sequence are each independently in a length of at least 1, 2, 3, 4, 5, 6, 7, 8, 9 or 10 amino acids.

In another specific embodiment, the first auxiliary sequence and the second auxiliary sequence are each independently selected from the group consisting of GSGS, EEGSGS, GSGSDDGSGS, GSGSEDGSGS, GSGSDEGSGS, EEAE, DDAD, TEEAEKL, TDDADKL, TEDAEKL, TDEADKL, GTEEAEKLG, EGTEEAEKLG, EGTDDADKLG, EGTEEADKLG, EGTDDAEKLG, EGTEDADKLG and EGTDEAEKLG, and sequences having at least 60%, 70%, 80%, or 90% identity thereto.

In a specific embodiment, the recombinant polypeptide has an amino acid sequence selected from the group consisting of SEQ ID NO: 4, SEQ ID NO: 15 and SEQ ID NO: 19.

In the second aspect, the present disclosure provides an isolated polynucleotide, encoding the recombinant polypeptide according to the first aspect of the present disclosure.

In a specific embodiment, the nucleic acid sequence of the polynucleotide is selected from the group consisting of SEQ ID NO: 5, SEQ ID NO: 16 and SEQ ID NO: 20.

In the third aspect, the present disclosure provides an expression vector, comprising the polynucleotide according to the second aspect of the present disclosure.

In the fourth aspect, the present disclosure provides a cell, comprising the polynucleotide according to the second aspect of the present disclosure or the expression vector according to the third aspect of the present disclosure.

In an embodiment, the cell is selected from the group consisting of a prokaryotic cell and a eukaryotic cell; for example, the prokaryotic cell is selected from *Escherichia coli* cells; for example, the eukaryotic cell is selected from the group consisting of yeast cells, insect cells, plant cells and mammalian cells.

In the fifth aspect, the present disclosure provides a method for producing a neurotoxin, comprising culturing the cell according to the fourth aspect of the present disclosure under conditions suitable for expression of the recombinant polypeptide.

In an embodiment, the method further comprises isolating the recombinant polypeptide expressed recombinantly, and cleaving the recombinant polypeptide with a protease.

In another embodiment, the method further comprises purifying the neurotoxin from cell culture.

In the sixth aspect, the present disclosure provides a pharmaceutical or cosmetic composition, comprising the neurotoxin produced by the method according to the fifth aspect of the present disclosure.

In the seventh aspect, the present disclosure provides use of the recombinant polypeptide according to the first aspect, the polynucleotide according to the second aspect, the expression vector according to the third aspect, or the cell according to the fourth aspect of the present disclosure in the manufacture of a neurotoxin, for example, the neurotoxin is selected from the group consisting of tetanus neurotoxin, diphtheria toxin, and botulinum neurotoxin.

In the present disclosure, the use of the recombinant polypeptide for the fusion expression of neurotoxin can produce a biologically active neurotoxin target protein rapidly and in high yield. The recombinant neurotoxin polypeptide designed in the present disclosure has an affinity purification tag, thereby facilitating manufacture by industrially conventional separation and purification methods, enabling ease of operation and reducing production cost. By combining the affinity tag used for purification with a protease cleavage site, the protease is enabled to preferentially and selectively cleave the polypeptide of the present disclosure, while avoiding cleavage within the neurotoxin protein sequence. It not only improves the cleavage efficiency of the fusion-expressed protein, but also enhances the structural integrity of the fusion-expressed neurotoxin protein, thereby increasing protein yield and biological activity. In addition, following protease cleavage, the resulting neurotoxin produced by fusion expression no longer contains exogenous amino acids or polypeptides, thereby improving safety.

In another aspect, the recombinant polypeptide constructed according to the present disclosure may, for example, lack the native linker sequence between the light chain and the heavy chain. On the one hand, the expressed recombinant neurotoxin protein remains in a non-toxic state prior to the protease cleavage step, thereby significantly improving safety during the production process. On the other hand, the absence of the native linker sequence avoids nonspecific cleavage thereof by cellular proteases, thereby substantially reducing protein heterogeneity and improving the homogeneity of the resulting neurotoxin protein. It further facilitates subsequent protein purification and significantly increases the yield, purity, and potency of the target protein.

### BRIEF DESCRIPTION OF DRAWINGS

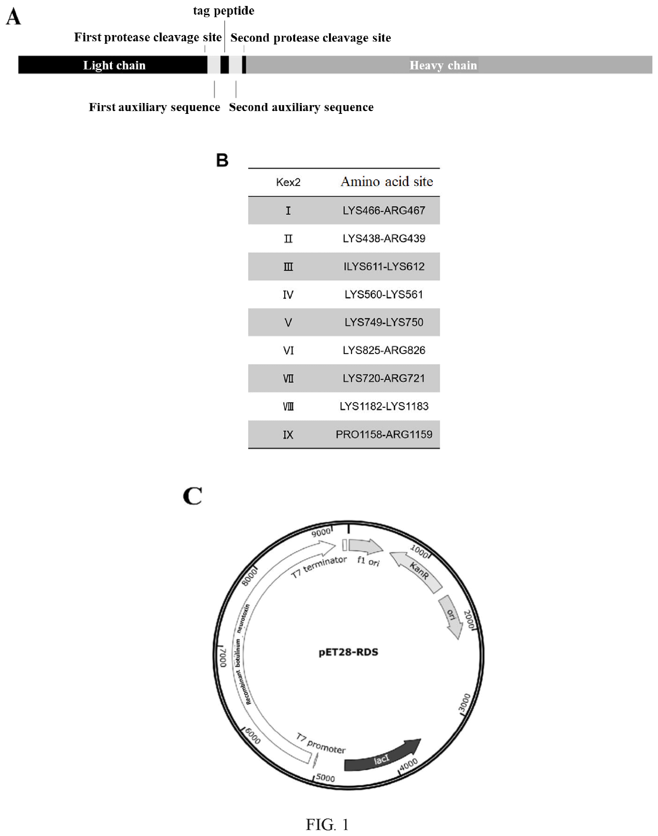

FIG. 1 is a schematic diagram of an exemplary recombinant botulinum toxin A protein structure and its expression vector. In FIG. 1, A is a schematic diagram of the recombinant botulinum toxin A protein structure, B is a schematic diagram of the Kex2 protease cleavage sites in the recombinant botulinum toxin A protein, and C is a schematic diagram of the expression vector plasmid inserted with the recombinant botulinum toxin gene sequence.

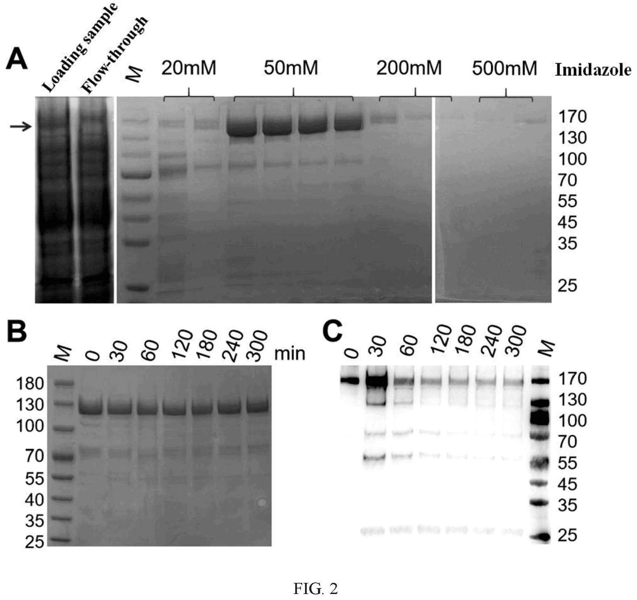

FIG. 2 is the results of nickel affinity chromatography purification of recombinant botulinum toxin A and analysis of enzyme cleavage stability. In FIG. 2, A is the electrophoresis results of nickel affinity chromatography purification, with arrows indicating the recombinant botulinum toxin; B is the electrophoresis results of Kex2 protease enzyme cleavage of purified samples; C is the Western blot results of the enzyme cleavage, with samples containing HIS tags before cleavage loaded at 100 ng, and other samples loaded at 3 µg. In the figures, M represents the protein molecular weight marker.

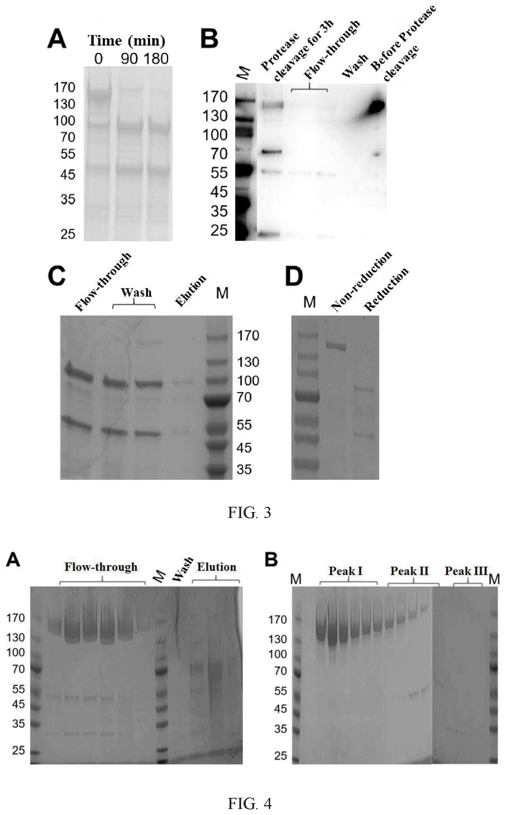

FIG. 3 is the results after recombinant botulinum toxin A is cleaved by enzyme and further purified by a second nickel column affinity chromatography. In FIG. 3, A is the reducing SDS-PAGE electrophoresis analysis after enzyme cleavage; B is the Western blot detection of enzyme cleavage efficiency, with HIS-tagged protein samples before cleavage loaded at 30 ng and other samples at 3 µg; C is the reducing SDS-PAGE electrophoresis after enzyme cleavage and the nickel column affinity chromatography; D is the electrophoresis of a recombinant protein comprising the native botulinum toxin 438-448 sequence processed at room temperature for 3 hours and run on SDS-PAGE. In the figures, M represents the protein molecular weight marker.
FIG. 4 is the results of anion exchange HQ column chromatography purification. In FIG. 4, A is the electrophoresis results of HQ column separation and purification; B is the electrophoresis results of Superdex 200 column separation and purification. In the figures, M represents the protein molecular weight marker.

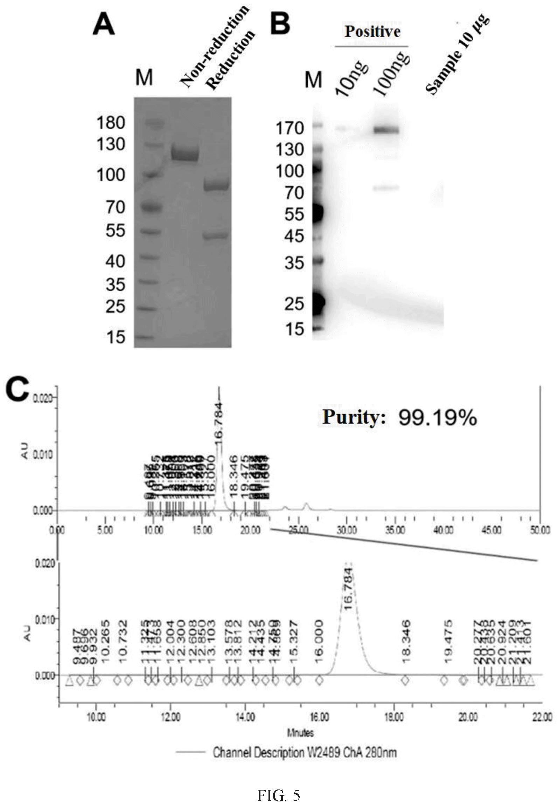

FIG. 5 shows the purity of the prepared recombinant botulinum toxin A and the detection results for HIS tag residues. In FIG. 5, A is the SDS-PAGE electrophoresis; B is the Western blot analysis ("positive" samples are purified, non-cleaved, HIS-tagged recombinant botulinum toxin protein); C is the HPLC chromatography. In the figures, M represents the protein molecular weight marker.

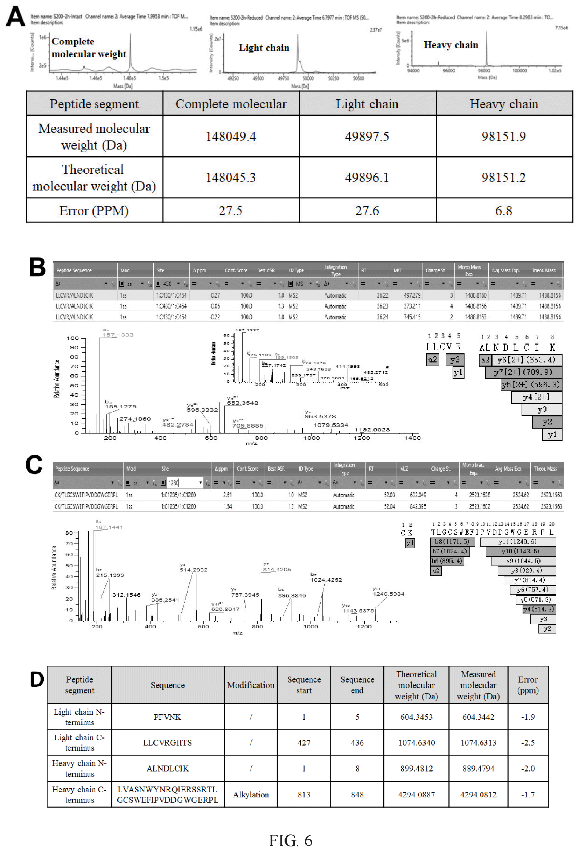

FIG. 6 is the results of mass spectrometry analysis of the primary structure of the prepared recombinant botulinum toxin A. In FIG. 6, A is the results of protein molecular weight detection; B is the results of C430/454 disulfide bond position detection; C is the results of C1235/1280 disulfide bond position detection; D is the detection results of the N- and C-terminal amino acid sequences of the protein.

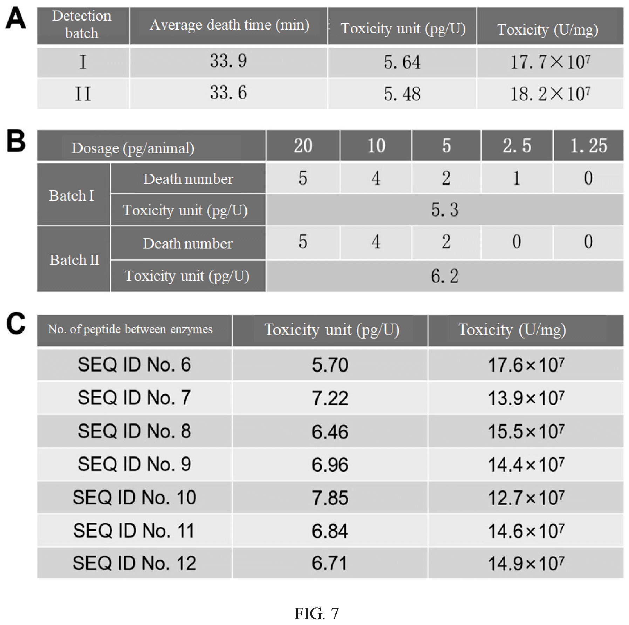

FIG. 7 is the results of toxicity measurement of the recombinant botulinum toxin A prepared by the present disclosure. In FIG. 7, A is the toxicity measurement results of tail vein injection of recombinant botulinum toxin A protein; B is the toxicity measurement results of intraperitoneal injection of recombinant botulinum toxin A protein; C is the toxicity measurement results after activation of recombinant botulinum toxin A proteins having different auxiliary sequences.

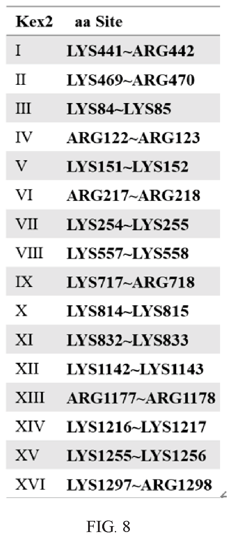

FIG. 8 is the Kex2 protease cleavage sites in recombinant botulinum toxin B protein prepared by the present disclosure.

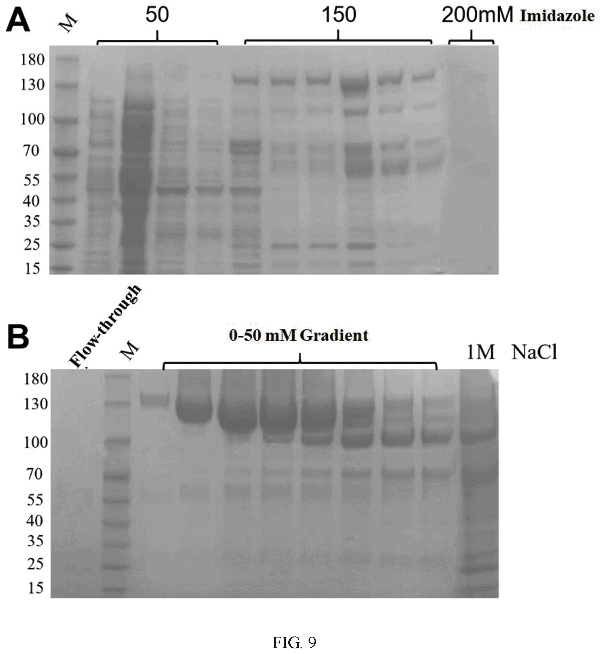

FIG. 9 is the results of recombinant botulinum toxin B after the first nickel affinity chromatography and anion exchange purification. In FIG. 9, A is the electrophoresis results of the first nickel affinity chromatography; B is the electrophoresis results of the first anion exchange HQ column chromatography.

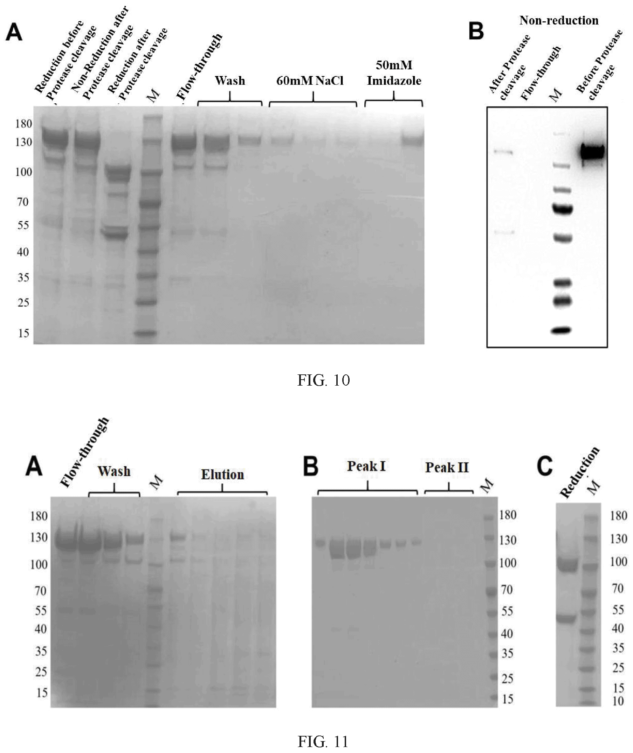

FIG. 10 is the results of recombinant botulinum toxin B after enzyme cleavage and the second nickel affinity chromatography purification. In FIG. 10, A shows the SDS-PAGE electrophoresis of the samples after Kex2 protease enzyme cleavage and second nickel affinity chromatography purification; B shows the Western blot result, with HIS-tagged samples before cleavage (0 min) loaded at 100 ng and other samples loaded at 3 µg. In the figures, M represents the protein molecular weight marker.
FIG. 11 is the results of recombinant botulinum toxin B after the second anion exchange column and gel filtration chromatography purification. In FIG. 11, A is the electrophoresis results of HQ column separation and purification; B is the electrophoresis results of Superdex 200 column separation and purification; C is the reducing SDS-PAGE electrophoresis of the prepared recombinant botulinum toxin B. In the figures, M represents the protein molecular weight marker.

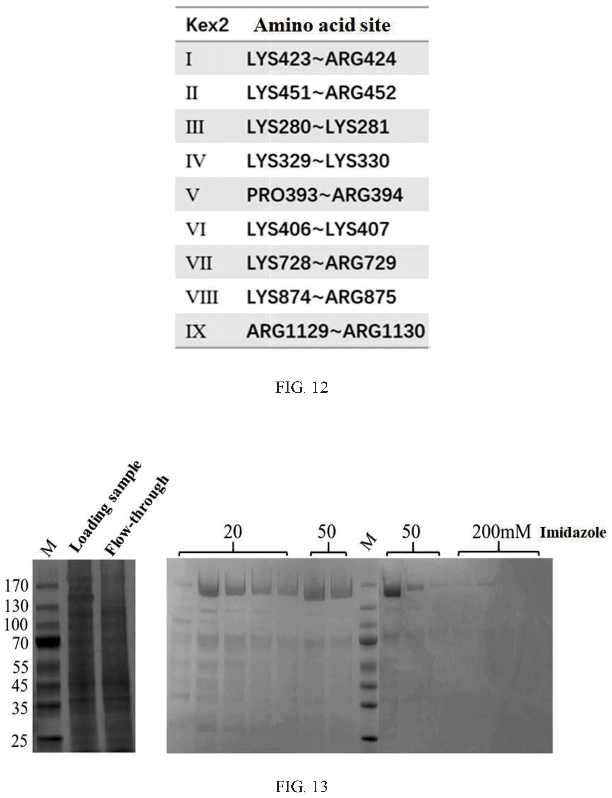

FIG. 12 shows the Kex2 protease cleavage sites in recombinant botulinum toxin E protein prepared in the present disclosure.
FIG. 13 is the nickel affinity chromatography purification result of recombinant botulinum toxin E. M represents the protein molecular weight marker.

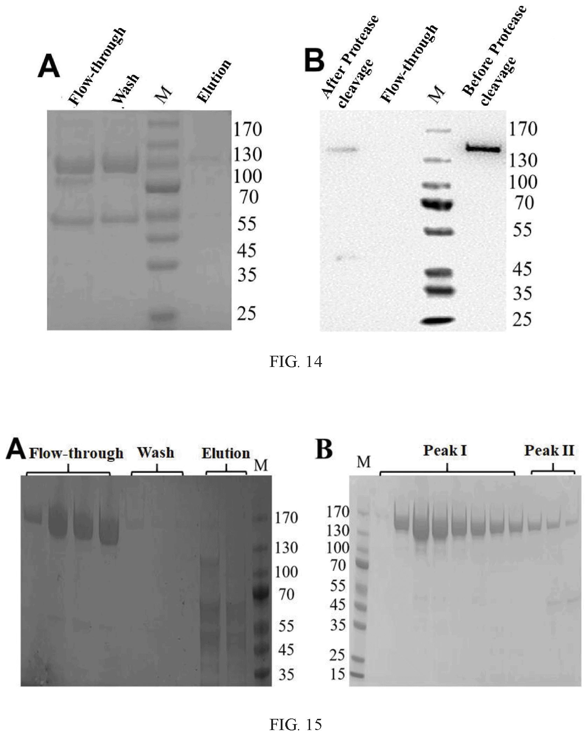

FIG. 14 is the results after recombinant botulinum toxin E is cleaved by enzyme and further purified by second nickel column affinity chromatography. In FIG. 14, A is the reducing SDS-PAGE electrophoresis results of nickel column affinity chromatography after enzyme cleavage; B is the Western blot detection of enzyme cleavage efficiency, with HIS-tagged protein samples before cleavage loaded at 30 ng and other samples loaded at 3 µg. M represents the protein molecular weight marker.
FIG. 15 is the anion exchange column and gel filtration chromatography purification results of recombinant botulinum toxin E. In FIG. 15, A is the electrophoresis results of HQ column separation and purification; B is the electrophoresis results of Superdex 200 column separation and purification. In the figures, M represents the protein molecular weight marker.

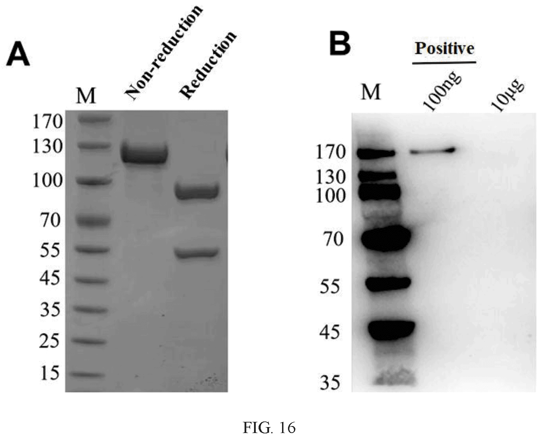

FIG. 16 shows the purity of the prepared recombinant botulinum toxin E and the detection results for HIS tag residues. In FIG. 16, A is the SDS-PAGE electrophoresis result; B is the Western blot analysis ("positive" samples are purified, non-cleaved, HIS-tagged recombinant botulinum toxin protein). In the figures, M represents the protein molecular weight marker.

### DETAILED DESCRIPTION

### Definition

Unless otherwise defined, the scientific and technical terms used herein have the meanings commonly understood by a person skilled in the art. The name and techniques used herein for cell and tissue culture, molecular biology, and protein and oligonucleotide or polynucleotide chemistry and hybridization are those well-known and commonly used in the art. Standard techniques are used for recombinant DNA, oligonucleotide synthesis, and tissue culture and transformation (e.g. electroporation and lipofection). Enzymatic reactions and purification techniques are performed according to the manufacturer's instructions, or according to methods commonly used in the art or as described herein. The above techniques and methods are generally carried out as described in various general and specific references well-known in the art and cited and discussed in the present specification. See, for example, Sambrook et al., Molecular Cloning: A Laboratory Manual (2nd ed., Cold Spring Harbor Laboratory Press, Cold Spring Harbor, New York, 1989). The name and laboratory procedures and techniques used herein in analytical chemistry, synthetic organic chemistry, and medical and pharmaceutical chemistry are those well-known and commonly used in the art.

As used herein, the term "neurotoxin" refers to a toxic substance that is destructive to nervous tissue. The neurotoxin may be a native neurotoxin or a recombinant neurotoxin, provided that it has neurotoxin activity. The light chain/heavy chain of a neurotoxin refers to different polypeptide chains of the neurotoxin linked by a disulfide bond. Generally, the light chain refers to the polypeptide chain having toxic activity, and the heavy chain refers to the polypeptide chain having binding activity.

As used herein, the term "linker peptide" refers to an exogenous amino acid sequence configured to connect the light chain or the heavy chain, or to connect both the light chain and the heavy chain such that the light chain and the heavy chain are located in the same polypeptide chain. The linker peptide comprises an affinity tag sequence and at least one protease cleavage site sequence selected from the group consisting of a first protease cleavage site sequence and a second protease cleavage site sequence. As used herein, the term "protease cleavage site sequence" refers to a sequence (also referred to as a recognition site) that can be recognized by a protease, in which or adjacent to which a specific cleavage site is present, and the protease cleaves the polypeptide chain at the cleavage site. As used herein, "cleavage at the junction" means that after protease cleavage, the linker peptide is completely removed from the light chain and/or the heavy chain.

Kex2 enzyme, also known as Kex2 protease, recombinant dibasic endopeptidase, or YSCF protease, specifically recognizes and cleaves peptide bonds at the carboxyl terminus of dibasic amino acids such as Arg-Arg, Lys-Arg, and Pro-Arg. Its recognition sequences include RR, KK, RK, or KR.

EK enzyme, also known as enterokinase, is a serine protease that specifically recognizes and cleaves the DDDDK sequence. Its recognition sequence includes DDDDK.

TEV protease, derived from tobacco etch virus (TEV), specifically recognizes the heptapeptide sequence EXXYXQG/S, and cleaves between the Gln and Gly/Ser residues. Commonly used recognition sequences thereof include ENLYFQG or ENLYFQS.

As used herein, the term "tag peptide" refers to a sequence added to a polypeptide chain for the purpose of separation and purification, which achieves protein purification through affinity adsorption and dissociation between biomolecules.

A polyhistidine tag (poly(His)-tag) refers to an amino acid sequence consisting of four, five, six, or more consecutive histidine residues, such as HHHHHH, HHHHHHH, or HHHHHHHH.

A FLAG tag is a short hydrophilic 8-amino-acid peptide DYKDDDDK. A streptavidin-binding tag (Strep-tag) is a short peptide tag that specifically binds to streptavidin, and can be divided into Strep-tag and Strep-tag II. A glutathione S-transferase tag (GST-tag) is a recombinant GST enzyme that can specifically bind to glutathione. These tag peptides having specific affinity capabilities can be used for specifically isolating polypeptides with such tags.

The first auxiliary sequence and the second auxiliary sequence, as used herein, refer to sequences located between the first protease cleavage site sequence (or the second protease cleavage site sequence) and the tag peptide. The provision of such auxiliary sequences can facilitate the protease cleavage site sequence, the tag peptide, and the prepared recombinant neurotoxin to better perform their respective functions.

By way of example, the present disclosure provides the following technical solutions:
1. A polypeptide for recombinantly expressing and producing a neurotoxin protein, comprising:
   a tag peptide for affinity purification;
   a first protease cleavage site comprising at least one amino acid and located on N-terminal side of the tag peptide; and
   a second protease cleavage site comprising at least one amino acid and located on C-terminal side of the tag peptide.
2. The polypeptide according to embodiment 1, wherein the tag peptide has a sequence of His-(Xm)n-His, wherein m and n are each independently selected from natural numbers.
3. The polypeptide according to embodiment 2, wherein m and n are each independently selected from natural numbers ranging from 1 to 10.
4. The polypeptide according to embodiment 2, wherein each Xm is independently selected from the group consisting of Lys, Met, Asp, Arg, Tyr, Glu and His.
5. The polypeptide according to embodiment 2, wherein the tag peptide is selected from the group consisting of HHHHHH, HHHHHHH and HHHHHHHH.
6. The polypeptide according to embodiment 1, wherein an amino acid of the first protease cleavage site is at least one selected from the group consisting of arginine and lysine.
7. The polypeptide according to embodiment 1, wherein the first protease cleavage site is selected from the group consisting of R, K, RR, KK, RK and KR.
8. The polypeptide according to embodiment 1, wherein the second protease cleavage site comprises at least one arginine or lysine.
9. The polypeptide according to embodiment 1, wherein the second protease cleavage site is selected from the group consisting of R, K, RR, KK, RK, KR and DDDDK.
10. The polypeptide according to embodiment 1, wherein the first protease cleavage site is a Kex2 protease cleavage site.
11. The polypeptide according to embodiment 1, wherein the first protease cleavage site and the second protease cleavage site are Kex2 protease cleavage sites.
12. The polypeptide according to embodiment 1, wherein the tag peptide further comprises a flexible sequence located at one or more positions at N-terminus and/or C-terminus of the tag peptide.
13. The polypeptide according to embodiment 1, wherein the flexible sequence comprises at least one glycine or serine.
14. The polypeptide according to embodiment 13, wherein the flexible sequence is selected from the group consisting of G, S, GS, SG, GG, SS, GGS, SGG, GSG and SGS.
15. The polypeptide according to embodiment13, wherein the flexible sequence is located between the tag peptide and the first protease cleavage site.
16. The polypeptide according to embodiment 13, wherein the flexible sequence is located between the tag peptide and the second protease cleavage site.
17. The polypeptide according to embodiment 1, wherein the polypeptide further comprises a first auxiliary sequence located between the tag peptide and the first protease cleavage site, or between the tag peptide and the second protease cleavage site.
18. The polypeptide according to embodiment 17, wherein the first auxiliary sequence is selected from the group consisting of GSGS, EEGSGS, GSGSDDGSGS, GSGSEDGSGS, GSGSDEGSGS, EEAE, DDAD, TEEAEKL, TDDADKL, TEDAEKL, TDEADKL, GTEEAEKLG, EGTEEAEKLG, EGTDDADKLG, EGTEEADKLG, EGTDDAEKLG, EGTEDADKLG and EGTDEAEKLG, and homologous sequences thereof having greater than 70% identity.
19. The polypeptide according to embodiment 1, wherein the polypeptide further comprises:
   a first auxiliary sequence located between the tag peptide and the first protease cleavage site; and
   a second auxiliary sequence located between the tag peptide and the second protease cleavage site.
20. The polypeptide according to embodiment 19, wherein the first auxiliary sequence and the second auxiliary sequence are independently selected from the group consisting of GSGS, EEGSGS, GSGSDDGSGS, GSGSEDGSGS, GSGSDEGSGS, EEAE, DDAD, TEEAEKL, TDDADKL, TEDAEKL, TDEADKL, GTEEAEKLG, EGTEEAEKLG, EGTDDADKLG, EGTEEADKLG, EGTDDAEKLG, EGTEDADKLG and EGTDEAEKLG, and homologous sequences thereof having greater than 70% identity.
21. The polypeptide according to embodiment 1, wherein the polypeptide is for connecting one heavy chain and one light chain constituting a neurotoxin; the light chain is connected to one end of the polypeptide and the heavy chain is connected to the other end of the polypeptide.
22. The polypeptide according to embodiment 1, wherein the polypeptide is for connecting one heavy chain and one light chain constituting a neurotoxin; the light chain is connected to N-terminus of the polypeptide and the heavy chain is connected to C-terminus of the polypeptide.
23. The polypeptide according to embodiment 1, wherein the polypeptide is for fusion expression of a neurotoxin; the light chain of the neurotoxin is connected to the N-terminus of the polypeptide and the heavy chain is connected to the C-terminus of the polypeptide.
24. The polypeptide according to embodiment 1, wherein the fusion expression is carried out in various expression systems selected from the group consisting of a prokaryotic expression system, a eukaryotic expression system, an insect baculovirus expression system, and a mammalian expression system.
25. The polypeptide according to embodiment 1, wherein the prokaryotic organism is *Escherichia coli*.
26. The polypeptide according to embodiment 25, wherein the *Escherichia coli* is selected from the group consisting of BL21(DE3), HMS174(DE3), pLysS and pLysE.
27. The polypeptide according to embodiment 1, wherein the polypeptide is for connecting one heavy chain and one light chain constituting a botulinum neurotoxin; the light chain of the botulinum neurotoxin is connected to the N-terminus of the polypeptide and the heavy chain is connected to the C-terminus of the polypeptide; or
   wherein the polypeptide is for connecting one heavy chain and one light chain constituting a tetanus neurotoxin; the light chain of the tetanus neurotoxin is connected to the N-terminus of the polypeptide and the heavy chain is connected to the C-terminus of the polypeptide.
28. The polypeptide according to embodiment 27, wherein the tag peptide is selected from the group consisting of HHHHHH, HHHHHHH and HHHHHHHH; the first protease cleavage site is a Kex2 protease cleavage site; and the second protease cleavage site is a Kex2 protease cleavage site or an enterokinase cleavage site.
29. The polypeptide according to embodiment 28, wherein the amino acid sequence of the first protease cleavage site is selected from the group consisting of R, K, RR, KK, RK and KR.
30. The polypeptide according to embodiment 28, wherein the amino acid sequence of the second protease cleavage site is selected from the group consisting of DDDDK, R, K, RR, KK, RK and KR.
31. The polypeptide according to embodiment 27, wherein the polypeptide further comprises a flexible sequence located at one or more positions at the N-terminus and/or the C-terminus of the tag peptide, and the flexible sequence is selected from the group consisting of G, S, GS, SG, GG, SS, GGS, SGG, GSG and SGS.
32. The polypeptide according to embodiment 27, wherein the polypeptide further comprises an auxiliary sequence located in at least one position of between the tag peptide and the first protease cleavage site and between the tag peptide and the second protease cleavage site.
33. The polypeptide according to embodiment 27, wherein the auxiliary sequence is selected from GSGS, EEGSGS, GSGSDDGSGS, GSGSEDGSGS, GSGSDEGSGS, EEAE, DDAD, TEEAEKL, TDDADKL, TEDAEKL, TDEADKL, GTEEAEKLG, EGTEEAEKLG, EGTDDADKLG, EGTEEADKLG, EGTDDAEKLG, EGTEDADKLG and EGTDEAEKLG, and homologous sequences thereof having greater than 70% identity.
34. A method for producing a neurotoxin protein by recombinant expression, comprising linking a light chain and a heavy chain of the neurotoxin to two ends of an artificial polypeptide sequence to form a fusion expression sequence, carrying out fusion expression in an expression system, and subjecting the expressed protein to protease cleavage, separation and purification to obtain a protein having the same sequence as that of a native neurotoxin or having greater than 70% identity thereto;
   wherein the artificial polypeptide comprises the polypeptide according to embodiment 1.
35. The method according to embodiment 34, wherein the expression system is selected from the group consisting of a prokaryotic expression system, a eukaryotic expression system, an insect baculovirus expression system and a mammalian expression system.
36. The method according to embodiment 34, wherein the expression system is *Escherichia coli*.
37. The method according to embodiment 34, wherein the first protease cleavage site of the polypeptide is a dibasic amino acid endopeptidase cleavage site, and the tag peptide is selected from the group consisting of FLAG, Myc, STREP, HA and a sequence having His-(Xm)n-His, wherein m and n are each independently selected from natural numbers.

### Examples

The technical solutions of the present disclosure are further described in detail in conjunction with examples below. However, these examples shall not be construed as limiting the present disclosure.

### Example 1: Construction of recombinant botulinum toxin A protein and engineered bacteria

FIG. 1A shows the structure of an exemplary recombinant botulinum toxin A protein of the present disclosure. Between the light chain and the heavy chain, a first protease cleavage site, a first auxiliary sequence, a tag peptide, a second auxiliary sequence, and a second protease cleavage site were included. A linker peptide for linking the light chain (SEQ ID NO: 1) and the heavy chain (SEQ ID NO: 2) of botulinum toxin A in the present example, linked the light chain at its N-terminus and linked the heavy chain at its C-terminus. The linker peptide, from the N-terminus to the C-terminus, had a structure as follows:
Kex2 protease cleavage site (KR) -- first auxiliary sequence (SEQ ID NO: 3) -- tag peptide (consisting of eight histidine residues) -- second auxiliary sequence (SEQ ID NO: 3)-Kex2 protease cleavage site (KR).

The recombinant polypeptide prepared in the present example (the amino acid sequence thereof is shown in SEQ ID NO: 4) had nine Kex2 protease cleavage sites, seven of which were also present in the native protein sequence of botulinum toxin, as shown in FIG. 1B.

According to the characteristics of the host *Escherichia coli*, the nucleotide coding sequence encoding the amino acid sequence of the recombinant botulinum toxin was designed and optimized, as shown in SEQ ID NO: 5. The nucleotide sequence was inserted into the pET-28 plasmid to obtain an expression vector pET-28-RDS, as shown in FIG. 1C. After sequencing verification confirmed that the sequence was correct, the expression vector was transformed into the host strain *E. coli* BL-21(DE3) (purchased from Thermo Fisher Scientific, USA).

### Example 2: Preparation of active recombinant botulinum toxin A protein

The engineered bacteria obtained in Example 1 were cultured in shake flasks, and expression of the recombinant botulinum toxin was induced with 0.3 mM IPTG. The fermentation broth was centrifuged to obtain bacterial cells, which were then disrupted by ultrasonication. The recombinant botulinum toxin A protein was preliminarily purified by nickel affinity chromatography. The non-native botulinum toxin amino acid sequences in the obtained protein were removed using Kex2 protease and carboxypeptidase B. Subsequently, multiple separation methods, including ion-exchange chromatography and size-exclusion chromatography, were carried out to obtain a target protein comprising only the native botulinum toxin A amino acid sequence with a purity greater than 99%. The specific separation and purification procedures are as follows:

### (1) Cell disruption

The bacterial cells obtained by centrifugation were resuspended in three times the bacterial cell volume of NaCl (20 g/L). The suspension was centrifuged at 4°C and 8000 rpm for 10 min, and the bacterial cells were collected. The collected bacterial cells were resuspended in nine times the bacterial cell volume of buffer (50 mM phosphate, 0.1 M NaCl, pH=7.0), and 5‰PMSF (100 mM) was added. Under low-temperature conditions, the bacterial cells were disrupted using a high-pressure homogenizer (Shanghai Hongli Technology Co., Ltd., 850 bar, twice). The lysate was centrifuged at 12,000 rpm for 40 min, and the supernatant was collected and filtered through a 0.45 µm filter to obtain a loading solution.

### (2) Nickel affinity chromatography

In a chromatography cabinet at 4°C, the loading solution was loaded to a pre-equilibrated nickel affinity chromatography column (Beyotime Biotechnology Co., Ltd.). The column was washed with five column volumes of equilibration buffer (50 mM phosphate, 0.1 M NaCl, pH=7.0) until no protein was detected in the flow-through. Stepwise gradient elution was then performed using an elution buffer of 20, 50, 200, and 500 mM imidazole in equilibration buffer (five column volumes for each gradient). The eluted fractions of each peak were collected separately, and the protein purity and content of fractions of each peak were analyzed by SDS-PAGE. The results showed that the recombinant botulinum toxin protein was mainly present in the fraction eluted with 50 mM imidazole, with relatively high purity, as shown in FIG. 2A. The fraction eluted with 50 mM imidazole was collected and subjected to 100-fold ultrafiltration with 50 mM phosphate buffer (pH=7.0). Kex2 protease was added at a ratio of 1:500 (enzyme : protein, w/w), and the mixture was incubated for 5 hours. Samples were taken at intervals for electrophoresis to evaluate the non-specific cleavage effect of the protease on the target protein. The inventor unexpectedly found that after 5 hours of protease cleavage, the amount of the target protein changed little, indicating that the non-specific cleavage effect of Kex2 protease on the target protein was very weak. Almost no significant cleavage in the recombinant polypeptide was observed at the Kex2 cleavage sites that naturally existed in the native botulinum toxin, as shown in FIG. 2B. The removal of the HIS tag was analyzed by Western blot. The results showed that with increasing incubation time, the HIS tag of the target protein gradually decreased. After 3 hours of protease cleavage (loading amount 3 µg), the rate of decrease was significantly reduced, and the band intensity was weaker than that of the pre-cleavage sample (loading amount 100 ng). This indicated that after incubation with the protease for 3 hours, more than 96% of the target protein had its HIS tag removed, as shown in FIG. 2C.

Thus, the present example verified that the scheme of removing the exogenous polypeptide by Kex2 protease cleavage was feasible.

### (3) Removal of the linker peptide

The eluate containing a large amount of the target protein after nickel affinity chromatography was collected and concentrated using a 50 kDa ultrafiltration centrifugal tube (Sartorius). Specifically, the protein eluate was concentrated to 1/6 of its original volume. The concentrated solution was then diluted 6 fold with cleavage buffer (50 mM phosphate, 0.1 M NaCl, pH=7.0), followed by a second round of concentration and dilution. The solution was subsequently concentrated to a protein concentration of approximately 2 mg/mL. Kex2 protease and carboxypeptidase B (Shanghai Yaxin Biotechnology Co., Ltd.) were added at a mass ratio of 1:500 (enzyme : protein, w/w). Cleavage buffer was supplemented to adjust the botulinum toxin protein concentration to 0.5 mg/mL, and protease cleavage was carried out at 25°C for 3 hours. The cleaved samples (loading solution for nickel column) were analyzed by reducing electrophoresis. The results showed that after cleavage, the protein bands were mainly observed at 100 kDa and 50 kDa, whereas the molecular weight of the protein without protease treatment was 150 kDa. This indicated that the proteases cleaved the target protein and further demonstrated that prior to cleavage, the protein presented as a single chain without toxicity, as shown in FIG. 3A. The removal effect of the HIS tag was further analyzed by Western blot using an anti-His antibody. Although the loading amount of the sample after 3-hour cleavage (3 µg) was 100 times that of the positive control protein (30 ng), the band intensity of the former was significantly weaker than that of the latter, indicating that more than 99% of the HIS tag was removed, as shown in FIG. 3B.

After completion of protease cleavage, the cleavage solution was subjected to a pre-equilibrated nickel affinity column once under low-temperature conditions (column equilibration buffer: 50 mM phosphate buffer, pH=7.0). The flow-through and wash fractions (three column volumes) were collected, and EDTA was added (a final concentration of 1 mM). The cleavage effect was analyzed by Western blot and SDS-PAGE. SDS-PAGE results showed that the cleaved protein was mainly present in the flow-through fraction, and only a small amount of the target protein band was observed in the elution fraction, indicating that most of the protein no longer carried the HIS tag. Western blot analysis further confirmed this result, as shown in FIG. 3B and FIG. 3C.

The recombinant protein of the present example did not contain the amino acid sequence of positions 439-448 of native botulinum toxin A. Experimental results showed that when a recombinant botulinum toxin A protein containing this native sequence was expressed in *E. coli*, it was cleaved into two chains even without the addition of protease, thereby showing toxicity, as shown in FIG. 3D. This resulted in the premature formation of the toxic form during the recombinant expression of botulinum toxin A, significantly increasing the biosafety risk during the preparation process.

### (4) Anion Exchange Chromatography

Under low-temperature conditions, the flow-through and wash fractions collected from nickel affinity chromatography after the protease cleavage were loaded onto a pre-equilibrated HQ anion exchange column (equilibration buffer: 50 mM phosphate buffer, pH=7.0). After the samples were loaded, the column was washed with 5-10 column volumes of equilibration buffer, and the flow-through peak was collected. Linear gradient elution was then performed with 0 mol/L to 1 mol/L NaCl over a total elution volume of 20 column volumes. The elution peak appeared after approximately five column volumes. The flow-through, wash, and elution fractions were collected, and the purity and content of recombinant botulinum toxin protein in each fraction were analyzed by SDS-PAGE. The results showed that the elution fraction mainly contained impurity proteins, while the botulinum toxin protein was primarily present in the flow-through fraction with a purity of approximately 95%, as shown in FIG. 4A. The collected flow-through fraction was concentrated to a protein concentration of 5 mg/mL using a 50 kDa ultrafiltration centrifugal tube (Sartorius), supplemented with 10% glycerol, and stored at -70°C for later use.

### (5) Superdex 200 gel filtration chromatography

Under low-temperature conditions, the concentrated sample was loaded onto a pre-equilibrated Superdex 200 column (Cytiva) (equilibration buffer: 50 mM phosphate, 0.1 M NaCl, pH=7.0). The column was eluted with equilibration buffer at a flow rate of 0.5 mL/min for two column volumes until no peak appeared. An elution peak was observed at approximately 23 minutes. The elution peaks were collected separately, and protein purity and content in each peak were analyzed by SDS-PAGE. The results showed that the botulinum toxin protein was mainly in elution peak I with essentially no impurity bands, while elution peaks II and III mainly contained impurity bands of approximately 55 kDa and 30 kDa, respectively, as shown in FIG. 4B.

### (6) Sample quantification and storage

Elution peak I from gel filtration chromatography was collected, added with 10% glycerol, and quantified by A280 measurement and the BCA method. The final protein concentration was adjusted to 1 mg/mL to 2 mg/mL. The purity of the final product and residual HIS tag were analyzed by SDS-PAGE, HPLC, and Western blot. The results showed that the sample presented a single band on SDS-PAGE, indicating electrophoretic purity. Western blot analysis showed that residual HIS tag was less than 1‰, and HPLC analysis indicated a purity of 99.19% (as shown in FIG. 5). The samples were aliquoted and stored at -70°C freezer.

The inventors also tested various linker peptide structures and other affinity tags such as FLAG, all of which achieved satisfactory expression, purification, and cleavage results, thereby obtaining recombinant botulinum toxin A protein as expected in the present disclosure.

### Example 3: Sequence identification of recombinant botulinum toxin A protein

Botulinum toxin A protein consists of two polypeptide chains linked by one interchain disulfide bond. The protein samples were treated in the absence or presence of the reducing agent TCEP, which cleaves the interchain disulfide bond and separates the two chains, thereby making the sample in a reduced state. After centrifugation at 12,000 g for 30 minutes, the protein samples were separated using an ACQUITY UPLC Protein BEH 300 C4 column and introduced into a Xevo G2-XS Q-TOF (Waters) mass spectrometer to determine the intact molecular weight and reduced molecular weight of the botulinum toxin A protein prepared in the present example. The mass spectrometry data were deconvoluted and analyzed using software UNIFI, and the specific data are shown in FIG. 6A. The results showed that the measured molecular weights of the full-length protein, light chain, and heavy chain were 148,049.4 Da (theoretical value: 148,045.3 Da), 49,897.5 Da (theoretical value: 49,896.1 Da), and 98,151.9 Da (theoretical value: 98,151.2 Da), respectively. The deviations from the theoretical molecular weights were all less than 28 ppm, indicating that the molecular weights of the botulinum toxin A protein prepared in this Example were consistent with the theoretical values. It demonstrated that the amino acid sequence of the obtained recombinant botulinum toxin A protein was identical to the designed sequence.

Disulfide bonds represent an important form of post-translational modification in proteins. Interchain or intrachain disulfide bonds are essential for maintaining the correct higher-order structure and biological activity of proteins. Nano liquid chromatography-tandem mass spectrometry (LC-MS/MS) was used to identify the intrachain and interchain disulfide bond pairing of the botulinum toxin A protein prepared in this Example. The nano liquid chromatography system and mass spectrometer were EASY-nLC 1200 and Thermo Orbitrap Q Exactive HF (Thermo Fisher Scientific, USA), respectively, and a C18 reversed-phase column was used for liquid chromatography. The mass spectrometry data were analyzed using software BioPharma Finder (V5.2). For each disulfide bond pair, abundant primary and secondary ion information was obtained. The mass deviation was 10 ppm for MS1 and 20 ppm for MS2. The results (FIG. 6B) showed that in the botulinum toxin A prepared by the present example, cysteine 429 of the light chain formed a disulfide bond with cysteine 6 of the heavy chain (corresponding to cysteines 430 and 454 of the native botulinum toxin protein), and cysteine 787 of the heavy chain formed a disulfide bond with cysteine 832 of the heavy chain (corresponding to the disulfide formed between cysteines 1235 and 1280 of the native botulinum toxin A protein). Detailed numerical data are shown in FIG. 6C. These results indicated that the botulinum toxin protein prepared in the present example contained one pair of interchain disulfide bonds and one pair of intrachain disulfide bonds, and that the connecting manner of the disulfide bonds was consistent with the theory.

The amino acid sequence constitutes the basis for protein function. The results shown in FIG. 6A suggested that the obtained recombinant botulinum toxin A protein had an amino acid sequence identical to the designed sequence. To further verify this result, the N-terminal and C-terminal sequences of the protein were analyzed. The protein sample prepared in the present example was denatured with guanidine hydrochloride, reduced, and digested with Glu-C and trypsin. The digested sample was separated using an ACQUITY UPLC Protein BEH 300 C4 column and introduced into a Xevo G2-XS Q-TOF (Waters) mass spectrometer for determination of the N-terminal and C-terminal amino acid sequences. The mass spectrometry data were analyzed using software UNIFI. The results showed that the N-terminal and C-terminal amino acid sequences of the light chain were PFVNK and LLCVRGIITS, respectively, and the N-terminal and C-terminal amino acid sequences of the heavy chain were ALNDLCIK and VDDGWGERPL, respectively, all of which were consistent with the theoretical sequences, as shown in FIG. 6D. It further indicated that after enzymatic cleavage, all exogenous amino acid sequences were completely removed.

### Example 4: Toxicity determination of recombinant botulinum toxin A protein

Five SPF-grade Kunming mice aged 26-30 days were used. Each mouse was injected via the tail vein with 0.1 mL of the recombinant botulinum toxin A product prepared in the present example at a concentration of 10 µg/mL. The average time to death (in minutes) was recorded. The sample toxicity was calculated according to the formula: Y = 17776578 - 868930 × t + 14,382 × t² - 78.90 × t³, where t represents the time to death (in minutes), and Y represents the corresponding toxicity calculated from the regression equation. Three batches were tested. The results showed that the LD₅₀ values (pg/25 g, U) of the recombinant botulinum toxin A measured in two independent determinations were 5.48 and 5.64, respectively. The average toxicity of the recombinant botulinum toxin A was therefore 5.56 pg/U. The average toxicity units (U) per mg of product to Kunming mice was 18 × 10⁷, as shown in FIG. 7A.

### Intraperitoneal Injection Method:

The samples were serially diluted. Using disposable sterile syringes, Each dilution was administered intraperitoneally to five female Kunming mice aged 26-30 days at 0.5mL per mouse. After injection, the onset of symptoms and mortality were observed at least once daily for four consecutive days, and the number of deaths was recorded. Three batches were tested. Based on the number of animal deaths within four days, the toxicity LD₅₀ of the sample was calculated according to the statistical Reed-Muench method. Calculation formula: $\begin{matrix}Dose corresponding to a \\ mortality rate of mice \\ greater than 50 %\end{matrix} \times 10 \left(\frac{\begin{matrix}Mortality percentage of \\ mice greater than 50 %\end{matrix} - 50 %}{\begin{matrix}Mortality percentage of \\ mice greater than 50 %\end{matrix} - \begin{matrix}Mortality percentage of \\ mice less than or equal to \\ 50 %\end{matrix}}\times lg \begin{matrix}Dilution \\ factor\end{matrix}\right)$

The results showed that the LD₅₀ values (pg/25 g, U) obtained in two independent measurements were 5.3 and 6.2, respectively. The average toxicity of the recombinant botulinum toxin A was therefore 5.75 pg/U. The average toxicity units (U) per mg of product to Kunming mice was 17.4 × 10! , as shown in FIG. 7B. The results obtained by the two toxicity determination methods were similar. The toxicity of the botulinum toxin A protein prepared in the present example was significantly higher than the minimum requirement of 1 × 10⁷ U/mg specified in the Chinese Pharmacopoeia. It further demonstrated that the amino acid sequence, disulfide bond positions, spatial folding, and other primary and higher-order structures of the botulinum toxin A protein were identical to those of the native protein.

The above data indicated that the linker peptide designed in the present disclosure ensured correct disulfide bond formation in botulinum toxin A, thereby enabling the recombinant botulinum toxin A protein to exhibit high biological activity. Based on the above examples, several additional auxiliary sequences (i.e., polypeptide sequences between the first protease cleavage site and the second protease cleavage site, also referred to herein as inter-enzyme polypeptides) were also employed. Corresponding expression clones were constructed, and the target proteins were prepared and activated. The effects of these auxiliary sequences and tag peptides on the biological activity of recombinant botulinum toxin (SEQ ID NO: 6 to SEQ ID NO: 12) were analyzed. The results showed that these flexible polypeptides all maintained the toxicity of recombinant botulinum toxin at a high level (12.7 × 10⁷ to 17.6 × 10⁷ U/mg), indicating that the use of these inter-enzyme polypeptides enabled the production of recombinant botulinum toxin A protein with high biological activity, as shown in FIG. 7C.

### Example 5: Preparation of recombinant botulinum toxin B protein

### (1) Construction of recombinant botulinum toxin B protein and engineered bacteria

The protein structure of the recombinant botulinum toxin B was similar to that of the recombinant botulinum toxin A protein described in Example 1, including a first protease cleavage site, a first auxiliary sequence, a tag peptide, a second auxiliary sequence, and a second protease cleavage site between the light chain and the heavy chain. A linker peptide for linking the light chain (SEQ ID NO: 13) and the heavy chain (SEQ ID NO: 14) of botulinum toxin B in the present example, linked the light chain at its N-terminus thereof and linked the heavy chain at its C-terminus. The structure of the linker peptide, from the N-terminus to the C-terminus, was as follows:

Kex2 protease cleavage site (KR) -- first auxiliary sequence (SEQ ID NO: 3) -- tag peptide (consisting of eight histidine residues) -- second auxiliary sequence (SEQ ID NO: 3)-Kex2 protease cleavage site (KR).

The recombinant botulinum toxin B polypeptide prepared in the present example (the amino acid sequence thereof is shown in SEQ ID NO: 15) had 16 Kex2 protease cleavage sites, 14 of which were also present in the native protein sequence of botulinum toxin, as shown in FIG. 8.

According to the characteristics of the host *Escherichia coli*, the nucleotide coding sequence encoding the amino acid sequence of the recombinant botulinum toxin B was designed and optimized, as shown in SEQ ID NO: 16. The nucleotide coding sequence was inserted into the pET-28 plasmid to obtain the expression vector pET-28-RDS-B. After sequencing verification confirmed that the sequence was correct, the expression vector was transformed into the host strain *E. coli* BL-21(DE3) (purchased from Thermo Fisher Scientific, USA).

### (2) Protein expression and cell disruption

The engineered bacteria expressing recombinant botulinum toxin B were cultured in shake flasks, and expression of recombinant botulinum toxin B was induced with 0.3 mM IPTG. The fermentation broth was centrifuged to obtain bacterial cells, which were resuspended in buffer at nine times the bacterial cells weight (50 mM phosphate, 0.1 M NaCl, pH 7.0). Under low-temperature conditions, the bacterial cells were disrupted using a high-pressure homogenizer (Shanghai Hongli Technology Co., Ltd., 800 bar to 900 bar, twice). The lysate was centrifuged at 12,000 rpm for 40 minutes, and the supernatant was collected and filtered through a 0.45 µm filter to obtain the loading solution.

### (3) First nickel affinity chromatography

The loading solution was loaded to a pre-equilibrated nickel affinity chromatography column (Ni-IDA Purose 6 Fast Flow, Qianchun Bio filling material). The column was washed with three column volumes of equilibration buffer (50 mM phosphate, 0.1 M NaCl, pH 7.0), and stepwise gradient elution was performed using an elution buffer prepared with equilibration buffer containing 50 mM, 150 mM, and 200 mM imidazole (five column volumes for each gradient). The eluted fractions of each peak were collected separately, and the protein purity and content of each fraction were analyzed by SDS-PAGE. The results showed that the recombinant botulinum toxin B protein was mainly present in the fraction eluted with 150 mM imidazole, as shown in FIG. 9A.

### (4) First anion exchange chromatography

The fraction eluted with 150 mM imidazole was collected and diluted three-fold with 2 mM Tris-HCl buffer (pH 7.0) to serve as the loading solution for anion exchange chromatography. The sample was loaded to a pre-equilibrated anion exchange column (Sepax 60Q). The column was washed with equilibration buffer (100 mM Tris-HCl, pH 7.0) until no protein was detected in the effluent. Linear gradient elution (0-100%) was performed using elution buffer (100 mM Tris-HCl, 50 mM NaCl, pH 7.0) for 5 column volumes (CV), and isocratic elution was performed using elution buffer (100 mM Tris-HCl, 1 M NaCl, pH 7.0) for 2-3 CV. The flow-through and elution fractions were collected, and the purity and content of recombinant botulinum toxin B in each fraction were analyzed by SDS-PAGE. The results showed that botulinum toxin B protein was mainly present in the linear elution fractions, as shown in FIG. 9B.

### (5) Removal of the linker peptide

The fractions containing relatively pure target protein from the anion exchange chromatography were collected and subjected to approximately 50-fold ultrafiltration with cleavage buffer (50 mM Tris-HCl, pH 7.0). The protein concentration was adjusted to approximately 0.5 mg/mL, and CaCl₂ was added to a final concentration of 2 mM. Kex2 protease and recombinant carboxypeptidase B (Shanghai Yaxin Biotechnology Co., Ltd.) were added at a mass ratio of 1:100 (enzyme : protein, w/w), and protease cleavage was carried out overnight at room temperature (25°C). The cleaved samples were analyzed by reducing electrophoresis. The results showed that after cleavage, the protein bands were mainly observed at 100 kDa and 50 kDa, whereas the molecular weight of the untreated sample was 150 kDa. It indicated that the proteases cleaved the target protein and further demonstrated that prior to cleavage, the protein existed as a single chain without toxicity, as shown in FIG. 10A.

### (6) Second nickel affinity chromatography

After protease cleavage was completed, the cleaved solution was subjected to a pre-equilibrated nickel affinity chromatography column (50 mM Tris-HCl, pH 7.0) once. The flow-through and wash fractions (three column volumes) were collected, and EDTA was added to a final concentration of 1 mM. Stepwise gradient elution was then performed using 60 mM NaCl and 50 mM imidazole in equilibration buffer (five column volumes for each gradient). The eluted fractions of each peak were collected separately. The cleavage effect was analyzed by Western blot and SDS-PAGE. SDS-PAGE results showed that the cleaved protein was mainly present in the flow-through fraction, and only a small amount of the target protein band was observed in the elution fraction, indicating that most of the protein no longer carried the HIS tag. Western blot analysis further confirmed this result, as shown in FIG. 10.

### (7) Second anion exchange chromatography

The flow-through and wash fractions collected after nickel affinity chromatography was loaded onto a pre-equilibrated anion exchange column (Sepax 60Q; equilibration buffer: 150 mM Tris-HCl, pH 7.0). After the sample was loaded, the column was washed with five column volumes of equilibration buffer, and the flow-through peak was collected. Isocratic elution was then performed with equilibration buffer containing 50 mM NaCl for five column volumes. The flow-through, wash, and elution fractions were collected, and the purity and content of recombinant botulinum toxin B protein in each fraction were analyzed by SDS-PAGE. The results showed that the elution fraction contained more impurity proteins, whereas botulinum toxin B protein was mainly present in the flow-through and wash fractions, as shown in FIG. 11A. The relatively pure protein from the flow-through and wash fractions were concentrated to approximately 5 mg/mL using a 50 kDa ultrafiltration centrifugal tube (Sartorius), supplemented with 10% glycerol, and stored at -70°C for later use.

### (8) Superdex 200 gel filtration chromatography

Under low-temperature conditions, the concentrated sample from the second anion exchange chromatography was loaded onto a pre-equilibrated Superdex 200 column (Cytiva) (equilibration buffer: 50 mM phosphate, 0.1 M NaCl, pH 7.0). The column was eluted with equilibration buffer at a flow rate of 0.5 mL/min for two column volumes until no peak appeared. An elution peak was observed at approximately 23 minutes. The elution peaks were collected separately, and protein purity and content in each peak were analyzed by SDS-PAGE. The results showed that botulinum toxin B protein was mainly in elution peak I, while elution peak II mainly contained an impurity band of approximately 30 kDa, as shown in FIG. 11B. The reducing electroporesis results of the final sample showed two bands corresponding to the light chain and heavy chain of recombinant botulinum toxin B respectively, indicating electrophoretic purity, as shown in FIG. 11C.

### Example 6: Preparation of recombinant botulinum toxin E protein

### (1) Construction of recombinant botulinum toxin E protein and engineered bacteria

The protein structure of the recombinant botulinum toxin E was similar to that of the recombinant botulinum toxin A protein described in Example 1, including a first protease cleavage site, a first auxiliary sequence, a tag peptide, a second auxiliary sequence, and a second protease cleavage site between the light chain and the heavy chain. A linker peptide for connecting the light chain (SEQ ID NO: 17) and the heavy chain (SEQ ID NO: 18) of botulinum toxin E in this Example, linked the light chain at its N-terminus and linked the heavy chain at its C-terminus. The structure of the linker peptide, from the N-terminus to the C-terminus, was as follows:
Kex2 protease cleavage site (KR) -- first auxiliary sequence (SEQ ID NO: 3) -- tag peptide (consisting of eight histidine residues) -- second auxiliary sequence (SEQ ID NO: 3)-Kex2 protease cleavage site (KR).

The recombinant polypeptide prepared in the present example (the amino acid sequence thereof is shown in SEQ ID NO: 19) had nine Kex2 protease cleavage sites, seven of which were also present in the native protein sequence of botulinum toxin, as shown in FIG. 12.

According to the characteristics of the host *Escherichia coli*, the nucleotide coding sequence encoding the amino acid sequence of the recombinant botulinum toxin E was designed and optimized, as shown in SEQ ID NO: 20. The nucleotide coding sequence was inserted into the pET-28 plasmid to obtain the expression vector pET-28-RDS-E. After sequencing verification confirmed that the sequence was correct, the expression vector was transformed into the host strain *E. coli* BL-21(DE3) (purchased from Thermo Fisher Scientific, USA).

### (2) Protein expression and cell disruption

The engineered bacteria expressing recombinant botulinum toxin E were cultured in shake flasks, and expression of recombinant botulinum toxin E was induced with 0.3 mM IPTG. The fermentation broth was centrifuged to obtain bacterial cells, which were resuspended in buffer at nine times the bacterial cells weight (50 mM phosphate, 0.1 M NaCl, pH 7.0). Under low-temperature conditions, the cells were disrupted using a high-pressure homogenizer (Shanghai Hongli Technology Co., Ltd., 800-900 bar, twice). The lysate was centrifuged at 12,000 rpm for 40 minutes, and the supernatant was collected and filtered through a 0.45 µm filter to obtain the loading solution.

### (3) First nickel affinity chromatography

The loading solution was loaded to a pre-equilibrated nickel affinity chromatography column (Beyotime Biotechnology Co., Ltd.). The column was washed with three column volumes of equilibration buffer (50 mM phosphate, 0.1 M NaCl, pH 7.0), and stepwise gradient elution was performed using an elution buffer prepared with equilibration buffer containing 20 mM, 50 mM, and 200 mM imidazole (five column volumes for each gradient). The eluted fractions of each peak were collected separately, and the protein purity and content of each fraction were analyzed by SDS-PAGE. The results showed that recombinant botulinum toxin E protein was mainly present in the fractions eluted with 20 mM and 50 mM imidazole, as shown in FIG. 13.

### (4) Removal of the linker peptide

The fractions containing the target protein from nickel affinity chromatography were collected and subjected to 100-fold ultrafiltration with 50 mM phosphate buffer (pH 7.0). Kex2 protease and recombinant carboxypeptidase B were added at a mass ratio of 1:100 (enzyme : protein, w/w), and protease cleavage was carried out at room temperature (25°C) for 5 hours. The cleaved sample (loading solution for nickel column) was then subjected to a second nickel affinity chromatography.

### (5) Second nickel affinity chromatography

After protease cleavage was completed, the cleaved solution was subjected to a pre-equilibrated nickel affinity chromatography column (equilibration buffer: 50 mM phosphate buffer, pH 7.0) once. The flow-through and wash fractions (three column volumes) were collected, and EDTA was added (final concentration of 1 mM). The fractions of each peak were collected separately, and the cleavage effect was analyzed by Western blot and SDS-PAGE. Reducing SDS-PAGE results showed that after cleavage, the protein was mainly present in the flow-through and wash fractions, with bands at approximately 100 kDa and 50 kDa, indicating that the proteases cleaved the target protein and that most of the protein no longer carried the HIS tag, as shown in FIG. 14A. Western blot analysis further confirmed this result, as shown in FIG. 14B.

### (6) Anion exchange chromatography

The flow-through and wash fractions collected after nickel affinity chromatography was loaded onto a pre-equilibrated HQ anion exchange column (equilibration buffer: 50 mM phosphate buffer, pH 7.0). After the sample was loaded, the column was washed with equilibration buffer until no protein was detected in the effluent, and the flow-through peak was collected. Elution was then performed with 1 mol/L NaCl. The flow-through, wash, and elution fractions were collected, and the purity and content of recombinant botulinum toxin E protein in each fraction were analyzed by SDS-PAGE. The results showed that the elution fraction mainly contained impurity proteins, whereas botulinum toxin E protein was primarily present in the flow-through fraction, as shown in FIG. 15A. The collected flow-through fraction was concentrated to 5 mg/mL using a 50 kDa ultrafiltration centrifugal tube (Sartorius), supplemented with 10% glycerol, and stored at -70°C for later use.

### (7) Superdex 200 gel filtration chromatography

The concentrated sample from anion exchange chromatography was loaded onto a pre-equilibrated Superdex 200 column (Cytiva) (50 mM phosphate, 0.1 M NaCl, pH 7.0). The column was eluted with equilibration buffer at a flow rate of 0.5 mL/min for two column volumes until no peak appeared. An elution peak was observed at approximately 23 minutes. The elution peaks were collected separately, and protein purity and content were analyzed by SDS-PAGE. The results showed that botulinum toxin E protein was mainly in elution peak I, while elution peak II mainly contained an impurity band of approximately 50 kDa, as shown in FIG. 15B.

### (8) Sample quantification and storage

Elution peak I from gel filtration chromatography was collected, supplemented with 10% glycerol, and quantified by A280 measurement and the BCA method. The final protein concentration was adjusted to 1 mg/mL to 2 mg/mL. The purity of the final product and residual HIS tag were analyzed by SDS-PAGE and WB. The results showed that the non-reducing electrophoresis of the samples presented a single band, indicating electrophoretic purity, while reducing electrophoresis showed two bands at approximately 100 kDa (heavy chain) and 50 kDa (light chain), as shown in FIG. 16A. WB analysis indicated that residual HIS tag was less than 1‰, as shown in FIG. 16B. The samples were aliquoted and stored at -70°C freezer.

The sequence information involved in the present disclosure is as follows:
SEQ ID NO: 1 
MPFVNKQFNYKDPVNGVDIAYIKIPNAGQMQPVKAFKIHNKIWVIPERDTFTNP
EEGDLNPPPEAKQVPVSYYDSTYLSTDNEKDNYLKGVTKLFERIYSTDLGRMLLTSIVRG
IPFWGGSTIDTELKVIDTNCINVIQPDGSYRSEELNLVIIGPSADIIQFECKSFGHEVLNLTRN
GYGSTQYIRFSPDFTFGFEESLEVDTNPLLGAGKFATDPAVTLAHELIHAGHRLYGIAINPN
RVFKVNTNAYYEMSGLEVSFEELRTFGGHDAKFIDSLQENEFRLYYYNKFKDIASTLNKA
KSIVGTTASLQYMKNVFKEKYLLSEDTSGKFSVDKLKFDKLYKMLTEIYTEDNFVKFFKV
LNRKTYLNFDKAVFKINIVPKVNYTIYDGFNLRNTNLAANFNGQNTEINNMNFTKLKNF
TGLFEFYKLLCVRGIITS
SEQ ID NO: 2 
ALNDLCIKVNNWDLFFSPSEDNFTNDLNKGEEITSDTNIEAAEENISLDLIQQYY
LTFNFDNEPENISIENLSSDIIGQLELMPNIERFPNGKKYELDKYTMFHYLRAQEFEHGKSR
IALTNSVNEALLNPSRVYTFFSSDYVKKVNKATEAAMFLGWVEQLVYDFTDETSEVSTTD
KIADITIIIPYIGPALNIGNMLYKDDFVGALIFSGAVILLEFIPEIAIPVLGTFALVSYIANKVLT
VQTIDNALSKRNEKWDEVYKYIVTNWLAKVNTQIDLIRKKMKEALENQAEATKAIINYQ
YNQYTEEEKNNINFNIDDLSSKLNESINKAMININKFLNQCSVSYLMNSMIPYGVKRLED
FDASLKDALLKYIYDNRGTLIGQVDRLKDKVNNTLSTDIPFQLSKYVDNQRLLSTFTEYI
KNIINTSILNLRYESNHLIDLSRYASKINIGSKVNFDPIDKNQIQLFNLESSKIEVILKNAIVY
NSMYENFSTSFWIRIPKYFNSISLNNEYTIINCMENNSGWKVSLNYGEIIWTLQDTQEIKQ
RVVFKYSQMINISDYINRWIFVTITNNRLNNSKIYINGRLIDQKPISNLGNIHASNNIMFKL
DGCRDTHRYIWIKYFNLFDKELNEKEIKDLYDNQSNSGILKDFWGDYLQYDKPYYMLNL
YDPNKYVDVNNVGIRGYMYLKGPRGSVMTTNIYLNSSLYRGTKFIIKKYASGNKDNIVR
NNDRVYINVVVKNKEYRLATNASQAGVEKILSALEIPDVGNLSQVVVMKSKNDQGITNK
 
CKMNLQDNNGNDIGFIGFHQFNNIAKLVASNWYNRQIERSSRTLGCSWEFIPVDDGWGE
RPL
SEQ ID NO: 3
   GTEEAEKLG
SEQ ID NO: 4 
MPFVNKQFNYKDPVNGVDIAYIKIPNAGQMQPVKAFKIHNKIWVIPERDTFTNP
EEGDLNPPPEAKQVPVSYYDSTYLSTDNEKDNYLKGVTKLFERIYSTDLGRMLLTSIVRG
IPFWGGSTIDTELKVIDTNCINVIQPDGSYRSEELNLVIIGPSADIIQFECKSFGHEVLNLTRN
GYGSTQYIRFSPDFTFGFEESLEVDTNPLLGAGKFATDPAVTLAHELIHAGHRLYGIAINPN
RVFKVNTNAYYEMSGLEVSFEELRTFGGHDAKFIDSLQENEFRLYYYNKFKDIASTLNKA
KSIVGTTASLQYMKNVFKEKYLLSEDTSGKFSVDKLKFDKLYKMLTEIYTEDNFVKFFKV
LNRKTYLNFDKAVFKINIVPKVNYTIYDGFNLRNTNLAANFNGQNTEINNMNFTKLKNF
TGLFEFYKLLCVRGIITSKRGTEEAEKLGHHHHHHHGTEEAEKLGKRALNDLCIKVNN
WDLFFSPSEDNFTNDLNKGEEITSDTNIEAAEENISLDLIQQYYLTFNFDNEPENISIENLSS
DIIGQLELMPNIERFPNGKKYELDKYTMFHYLRAQEFEHGKSRIALTNSVNEALLNPSRV
YTFFSSDYVKKVNKATEAAMFLGWVEQLVYDFTDETSEVSTTDKIADITIIIPYIGPALNIG
NMLYKDDFVGALIFSGAVILLEFIPEIAIPVLGTFALVSYIANKVLTVQTIDNALSKRNEKW
DEVYKYIVTNWLAKVNTQIDLIRKKMKEALENQAEATKAIINYQYNQYTEEEKNNINFNI
DDLSSKLNESINKAMININKFLNQCSVSYLMNSMIPYGVKRLEDFDASLKDALLKYIYDN
RGTLIGQVDRLKDKVNNTLSTDIPFQLSKYVDNQRLLSTFTEYIKNIINTSILNLRYESNHL
IDLSRYASKINIGSKVNFDPIDKNQIQLFNLESSKIEVILKNAIVYNSMYENFSTSFWIRIPK
YFNSISLNNEYTIINCMENNSGWKVSLNYGEIIWTLQDTQEIKQRVVFKYSQMINISDYIN
RWIFVTITNNRLNNSKIYINGRLIDQKPISNLGNIHASNNIMFKLDGCRDTHRYIWIKYFNL
FDKELNEKEIKDLYDNQSNSGILKDFWGDYLQYDKPYYMLNLYDPNKYVDVNNVGIRG
YMYLKGPRGSVMTTNIYLNSSLYRGTKFIIKKYASGNKDNIVRNNDRVYINVVVKNKEY
RLATNASQAGVEKILSALEIPDVGNLSQVVVMKSKNDQGITNKCKMNLQDNNGNDIGFI
GFHQFNNIAKLVASNWYNRQIERSSRTLGCSWEFIPVDDGWGERPL
SEQ ID NO: 5 ATGCCGTTTGTGAACAAACAGTTCAACTATAAAGATCCGGTGAACGGTGTTG 
ATATCGCCTATATCAAAATTCCGAATGCAGGTCAGATGCAGCCGGTTAAAGCCTTTAAA
ATCCATAACAAAATTTGGGTGATTCCGGAACGTGATACCTTTACCAATCCGGAAGAAG
GTGATCTGAATCCGCCTCCGGAAGCAAAACAGGTTCCGGTTAGCTATTATGATAGCAC
CTATCTGAGCACCGATAACGAGAAAGATAACTATCTGAAAGGTGTGACCAAACTGTTT
GAACGCATTTATAGTACCGATCTGGGTCGTATGCTGCTGACCAGCATTGTTCGTGGTAT
TCCGTTTTGGGGTGGTAGCACCATTGATACCGAACTGAAAGTTATTGACACCAACTGC
ATTAATGTGATTCAGCCGGATGGTAGCTATCGTAGCGAAGAACTGAATCTGGTTATTAT
TGGTCCGAGCGCAGATATCATTCAGTTTGAATGTAAAAGCTTTGGCCACGAAGTTCTG
AATCTGACCCGTAATGGTTATGGTAGTACCCAGTATATTCGTTTCAGTCCGGATTTTACC
TTTGGCTTTGAAGAAAGCCTGGAAGTTGATACAAATCCGCTGTTAGGTGCAGGTAAAT
TTGCAACCGATCCGGCAGTTACCCTGGCACATGAACTGATTCATGCCGGTCATCGTCT
GTATGGTATTGCAATTAATCCGAACCGTGTGTTCAAAGTGAATACCAACGCATATTATG
AAATGAGCGGTCTGGAAGTGTCATTTGAAGAACTGCGTACCTTTGGTGGTCATGATGC
CAAATTTATCGATAGCCTGCAAGAAAATGAATTTCGCCTGTACTACTATAACAAATTCA
AGGATATTGCGAGCACCCTGAATAAAGCCAAAAGCATTGTTGGCACCACCGCAAGCC
TGCAGTATATGAAAAATGTGTTTAAAGAAAAATATCTGCTGAGCGAAGATACCAGCGG
TAAATTTAGCGTTGACAAACTGAAATTCGATAAACTGTACAAGATGCTGACCGAGATT
TATACCGAAGATAACTTCGTGAAGTTTTTCAAAGTGCTGAACCGCAAAACCTACCTGA
ACTTTGATAAAGCCGTGTTCAAAATCAACATCGTGCCGAAAGTGAACTATACCATCTAT
GATGGTTTTAACCTGCGCAATACCAATCTGGCAGCAAACTTTAATGGTCAGAACACCG
AAATCAACAACATGAACTTTACCAAACTGAAGAACTTCACCGGTCTGTTCGAATTTTA
CAAACTGCTGTGTGTTCGTGGCATTATTACCAGCAAACGAGGAACAGAAGAGGCTGA
AAAACTAGGGCACCACCATCATCATCACCACCACGGCACCGAAGAGGCGGAGAAGCT
GGGTAAACGTGCCCTGAATGATCTGTGCATTAAGGTGAATAATTGGGACCTGTTTTTC
AGCCCGAGTGAAGATAATTTCACCAACGATCTGAACAAAGGCGAAGAAATTACCAGC
GATACCAATATTGAAGCAGCCGAAGAAAACATTAGCCTGGATCTGATTCAGCAGTATTA
TCTGACCTTCAACTTCGATAATGAGCCGGAAAATATCAGCATTGAAAACCTGAGCAGC
GATATTATTGGCCAGCTGGAACTGATGCCGAATATTGAACGTTTTCCGAACGGCAAAA
AGTACGAGCTGGATAAATACACCATGTTCCATTATCTGCGTGCCCAAGAATTTGAACAT
GGTAAAAGCCGTATTGCACTGACCAATAGCGTTAATGAAGCACTGCTGAACCCGAGCC
GTGTTTATACCTTTTTCAGCAGCGATTATGTGAAGAAGGTTAACAAAGCAACCGAAGC
 
AGCAATGTTTTTAGGTTGGGTTGAACAGCTGGTGTATGATTTCACCGATGAAACCAGC
GAAGTTAGCACCACCGATAAAATTGCAGATATTACCATCATCATCCCGTATATCGGTCC
GGCACTGAATATTGGCAATATGCTGTATAAAGACGATTTTGTGGGTGCCCTGATTTTTA
GCGGTGCAGTTATTCTGCTGGAATTTATTCCGGAAATTGCCATTCCGGTTCTGGGCACC
TTTGCACTGGTGAGCTATATTGCAAATAAAGTTCTGACCGTGCAGACCATCGATAATGC
ACTGAGCAAACGTAACGAAAAATGGGATGAAGTGTACAAGTATATCGTGACCAATTG
GCTGGCAAAAGTTAACACCCAGATTGATCTGATCCGTAAAAAGATGAAAGAAGCCCT
GGAAAATCAGGCAGAAGCAACCAAAGCCATTATCAACTATCAGTATAACCAGTACACC
GAGGAAGAGAAGAATAACATCAACTTCAACATCGATGATCTGAGCAGCAAGCTGAAT
GAAAGCATCAACAAAGCCATGATTAACATTAACAAATTTCTGAACCAGTGCAGCGTGA
GCTATCTGATGAATAGCATGATTCCGTATGGTGTGAAACGTCTGGAAGATTTTGATGCA
AGCCTGAAAGATGCTCTGCTGAAATATATCTATGATAATCGTGGCACCCTGATTGGTCA
GGTTGATCGTCTGAAAGATAAAGTGAACAACACCCTGAGTACCGATATTCCTTTTCAG
CTGAGCAAATATGTGGATAATCAGCGTCTGCTGTCAACCTTTACCGAATACATTAAGAA
CATCATCAACACCAGCATTCTGAACCTGCGTTATGAAAGCAATCATCTGATCGATCTGT
CTCGCTATGCAAGCAAAATCAATATAGGTAGCAAGGTGAACTTCGACCCGATTGACAA
AAATCAGATACAGCTGTTTAATCTGGAAAGCAGCAAAATCGAAGTGATCCTGAAAAA
CGCCATTGTGTATAACAGCATGTATGAGAATTTCTCGACCAGCTTTTGGATTCGCATTC
CGAAATACTTTAATAGCATCAGCCTGAACAACGAGTACACCATTATTAACTGCATGGAA
AACAATAGCGGTTGGAAAGTGAGCCTGAATTATGGTGAGATTATTTGGACCCTGCAGG
ATACCCAAGAAATCAAACAGCGTGTGGTTTTCAAATACAGCCAGATGATTAATATCAG
CGACTATATCAACCGCTGGATTTTTGTGACCATTACCAATAATCGCCTGAATAACAGCA
AGATCTATATTAACGGTCGCCTGATTGATCAGAAACCGATTAGTAATCTGGGCAATATT
CATGCGAGCAACAACATCATGTTTAAACTGGATGGTTGTCGTGATACCCATCGTTATAT
TTGGATCAAGTACTTCAACCTGTTCGATAAAGAGCTGAACGAAAAAGAAATTAAAGA
TCTGTATGATAACCAGAGCAACAGCGGTATTCTGAAGGATTTTTGGGGAGATTATCTGC
AGTATGACAAACCGTATTATATGCTGAACCTGTACGACCCGAACAAATATGTTGATGTG
AACAATGTGGGTATCCGTGGCTATATGTACCTGAAAGGTCCGCGTGGTAGCGTTATGAC
CACAAACATTTATCTGAATAGCAGCCTGTATCGCGGAACCAAATTCATCATTAAAAAGT
ATGCCAGCGGCAACAAGGATAATATTGTGCGTAATAATGATCGCGTGTACATTAACGTT
GTGGTGAAGAATAAAGAATATCGCCTGGCAACCAATGCAAGCCAGGCAGGCGTTGAG
 
AAAATTCTGAGCGCACTGGAAATTCCGGATGTTGGTAATCTGAGCCAGGTTGTTGTGA
TGAAAAGCAAAAATGATCAGGGCATCACCAACAAGTGCAAAATGAATCTGCAGGACA
ATAACGGCAACGATATTGGTTTTATTGGCTTCCACCAGTTCAACAATATTGCGAAACTG
GTTGCAAGCAATTGGTATAATCGTCAGATTGAACGTAGCAGTCGTACCCTGGGTTGTA
GCTGGGAATTTATCCCTGTGGATGATGGTTGGGGTGAACGTCCGCTG
SEQ ID NO: 6
   HHHHHHHH
SEQ ID NO: 7
   GGSHHHHHHHHGGS
SEQ ID NO: 8
   GTEEAEKLGHHHHHHHH
SEQ ID NO: 9
   HHHHHHHHGTEEAEKLG
SEQ ID NO: 10
   GSGSEEGSGEGTEEAEKLGHHHHHHHHGSGSEEGSGEGTEEAEKLG
SEQ ID NO: 11
   DDADHHHHHHHHDDAD
SEQ ID NO: 12
   TEDAEKLHHHHHHHHTEDAEKL
SEQ ID NO: 13 
MPVTINNFNYNDPIDNNNIIMMEPPFARGTGRYYKAFKITDRIWIIPERYTFGYKP
EDFNKSSGIFNRDVCEYYDPDYLNTNDKKNIFLQTMIKLFNRIKSKPLGEKLLEMIINGIP
YLGDRRVPLEEFNTNIASVTVNKLISNPGEVERKKGIFANLIIFGPGPVLNENETIDIGIQNH
FASREGFGGIMQMKFCPEYVSVFNNVQENKGASIFNRRGYFSDPALILMHELIHVLHGLY
GIKVDDLPIVPNEKKFFMQSTDAIQAEELYTFGGQDPSIITPSTDKSIYDKVLQNFRGIVDR
LNKVLVCISDPNININIYKNKFKDKYKFVEDSEGKYSIDVESFDKLYKSLMFGFTETNIAE
 
NYKIKTRASYFSDSLPPVKIKNLLDNEIYTIEEGFNISDKDMEKEYRGQNKAINKQAYEEIS
KEHLAVYKIQMCKSV
SEQ ID NO: 14 
APGICIDVDNEDLFFIADKNSFSDDLSKNERIEYNTQSNYIENDFPINELILDTDLI
SKIELPSENTESLTDFNVDVPVYEKQPAIKKIFTDENTIFQYLYSQTFPLDIRDISLTSSFDDA
LLFSNKVYSFFSMDYIKTANKVVEAGLFAGWVKQIVNDFVIEANKSNTMDKIADISLIVP
YIGLALNVGNETAKGNFENAFEIAGASILLEFIPELLIPVVGAFLLESYIDNKNKIIKTIDNA
LTKRNEKWSDMYGLIVAQWLSTVNTQFYTIKEGMYKALNYQAQALEEIIKYRYNIYSEK
EKSNINIDFNDINSKLNEGINQAIDNINNFINGCSVSYLMKKMIPLAVEKLLDFDNTLKKN
LLNYIDENKLYLIGSAEYEKSKVNKYLKTIMPFDLSIYTNDTILIEMFNKYNSEILNNIILNL
RYKDNNLIDLSGYGAKVEVYDGVELNDKNQFKLTSSANSKIRVTQNQNIIFNSVFLDFSV
SFWIRIPKYKNDGIQNYIHNEYTIINCMKNNSGWKISIRGNRIIWTLIDINGKTKSVFFEYNI
REDISEYINRWFFVTITNNLNNAKIYINGKLESNTDIKDIREVIANGEIIFKLDGDIDRTQFI
WMKYFSIFNTELSQSNIEERYKIQSYSEYLKDFWGNPLMYNKEYYMFNAGNKNSYIKLK
KDSPVGEILTRSKYNQNSKYINYRDLYIGEKFIIRRKSNSQSINDDIVRKEDYIYLDFFNLN
QEWRVYTYKYFKKEEEKLFLAPISDSDEFYNTIQIKEYDEQPTYSCQLLFKKDEESTDEIG
LIGIHRFYESGIVFEEYKDYFCISKWYLKEVKRKPYNLKLGCNWQFIPKDEGWTE
SEQ ID NO: 15 
MPVTINNFNYNDPIDNNNIIMMEPPFARGTGRYYKAFKITDRIWIIPERYTFGYKP
EDFNKSSGIFNRDVCEYYDPDYLNTNDKKNIFLQTMIKLFNRIKSKPLGEKLLEMIINGIP
YLGDRRVPLEEFNTNIASVTVNKLISNPGEVERKKGIFANLIIFGPGPVLNENETIDIGIQNH
FASREGFGGIMQMKFCPEYVSVFNNVQENKGASIFNRRGYFSDPALILMHELIHVLHGLY
GIKVDDLPIVPNEKKFFMQSTDAIQAEELYTFGGQDPSIITPSTDKSIYDKVLQNFRGIVDR
LNKVLVCISDPNININIYKNKFKDKYKFVEDSEGKYSIDVESFDKLYKSLMFGFTETNIAE
NYKIKTRASYFSDSLPPVKIKNLLDNEIYTIEEGFNISDKDMEKEYRGQNKAINKQAYEEIS
KEHLAVYKIQMCKSVKRGTEEAEKLGHHHHHHHGTEEAEKLGKRAPGICIDVDNEDL
FFIADKNSFSDDLSKNERIEYNTQSNYIENDFPINELILDTDLISKIELPSENTESLTDFNVDV
PVYEKQPAIKKIFTDENTIFQYLYSQTFPLDIRDISLTSSFDDALLFSNKVYSFFSMDYIKTA
NKVVEAGLFAGWVKQIVNDFVIEANKSNTMDKIADISLIVPYIGLALNVGNETAKGNFEN
 
AFEIAGASILLEFIPELLIPVVGAFLLESYIDNKNKIIKTIDNALTKRNEKWSDMYGLIVAQ
WLSTVNTQFYTIKEGMYKALNYQAQALEEIIKYRYNIYSEKEKSNINIDFNDINSKLNEGI
NQAIDNINNFINGCSVSYLMKKMIPLAVEKLLDFDNTLKKNLLNYIDENKLYLIGSAEYE
KSKVNKYLKTIMPFDLSIYTNDTILIEMFNKYNSEILNNIILNLRYKDNNLIDLSGYGAKVE
VYDGVELNDKNQFKLTSSANSKIRVTQNQNIIFNSVFLDFSVSFWIRIPKYKNDGIQNYIH
NEYTIINCMKNNSGWKISIRGNRIIWTLIDINGKTKSVFFEYNIREDISEYINRWFFVTITNN
LNNAKIYINGKLESNTDIKDIREVIANGEIIFKLDGDIDRTQFIWMKYFSIFNTELSQSNIEE
RYKIQSYSEYLKDFWGNPLMYNKEYYMFNAGNKNSYIKLKKDSPVGEILTRSKYNQNSK
YINYRDLYIGEKFIIRRKSNSQSINDDIVRKEDYIYLDFFNLNQEWRVYTYKYFKKEEEKL
FLAPISDSDEFYNTIQIKEYDEQPTYSCQLLFKKDEESTDEIGLIGIHRFYESGIVFEEYKDY
FCISKWYLKEVKRKPYNLKLGCNWQFIPKDEGWTE
SEQ ID NO: 16 
ATGCCCGTAACAATAAATAACTTTAATTATAATGACCCGATTGATAACAACAAT
ATCATTATGATGGAACCGCCATTTGCGCGTGGTACTGGTCGTTATTACAAGGCCTTCAA
AATCACCGACCGTATTTGGATTATTCCGGAACGTTACACCTTCGGTTATAAGCCGGAGG
ACTTCAATAAAAGCAGCGGTATTTTCAACCGTGATGTGTGTGAATACTACGACCCGGA
TTATCTGAATACGAATGACAAAAAGAACATTTTCCTGCAGACGATGATTAAGCTGTTTA
ACCGCATTAAATCCAAACCGCTGGGTGAAAAGTTGCTGGAGATGATCATTAACGGTAT
TCCGTACCTTGGAGATCGCCGTGTTCCGCTGGAGGAATTCAATACCAATATCGCGTCC
GTTACCGTTAATAAGTTGATCAGCAATCCGGGGGAAGTGGAGCGCAAAAAAGGTATTT
TCGCTAACCTGATAATCTTCGGCCCAGGTCCGGTGCTGAACGAGAATGAAACCATTGA
CATCGGCATTCAGAACCACTTTGCGTCTAGAGAAGGATTTGGCGGTATCATGCAGATG
AAATTCTGCCCGGAATACGTGTCTGTCTTCAATAACGTGCAGGAAAACAAGGGTGCC
AGCATCTTCAACCGTCGCGGTTATTTCAGCGACCCGGCGCTGATTCTGATGCATGAAC
TCATTCATGTTCTGCATGGTTTGTACGGCATCAAGGTAGACGATCTACCGATCGTGCCG
AATGAAAAGAAATTCTTCATGCAGAGCACCGATGCGATTCAGGCCGAAGAGTTGTAC
ACCTTTGGTGGTCAAGATCCGTCCATCATCACCCCGAGCACGGACAAGTCGATCTACG
ATAAAGTGCTGCAAAACTTTCGTGGCATCGTTGACCGCCTGAACAAGGTGCTGGTTTG
CATTAGCGACCCGAATATCAACATCAACATTTACAAGAATAAGTTCAAGGACAAGTAC
AAGTTCGTCGAAGATTCCGAGGGTAAATACTCTATCGACGTCGAGAGCTTCGATAAAC
 
TGTACAAGTCTCTGATGTTCGGTTTTACCGAAACCAACATCGCGGAAAATTACAAGAT
TAAGACCCGTGCTAGCTACTTCTCTGACAGCCTGCCGCCTGTGAAAATCAAAAACTTG
CTGGACAACGAAATCTATACCATTGAGGAAGGATTTAATATTTCCGATAAAGATATGGA
GAAGGAGTACCGTGGCCAAAACAAAGCGATCAATAAACAGGCCTATGAAGAGATCAG
CAAAGAACATCTGGCAGTTTATAAGATACAGATGTGCAAAAGTGTGAAGCGCGGTAC
CGAAGAGGCCGAGAAGTTGGGCCACCATCATCACCACCACCACGGCACCGAAG
AGGCGGAAAAGTTGGGTAAACGCGCGCCAGGTATTTGCATCGATGTTGATAACGAGG
ATCTGTTTTTCATAGCGGATAAAAATAGCTTCAGCGACGATCTGAGCAAGAATGAACG
TATCGAGTATAACACGCAGAGCAACTATATTGAGAACGACTTCCCGATTAACGAGTTAA
TCCTGGACACCGACCTCATCTCCAAGATCGAATTACCGAGCGAGAATACTGAGAGCCT
AACTGATTTTAACGTGGATGTTCCGGTATATGAGAAGCAACCGGCAATTAAGAAGATC
TTCACCGACGAGAACACCATTTTCCAGTATCTTTACAGCCAAACGTTTCCGCTCGATAT
CCGTGATATCTCTCTGACCAGCAGCTTTGACGACGCGCTGCTGTTCTCGAACAAGGTT
TACAGCTTTTTCAGCATGGATTACATCAAGACTGCGAACAAGGTCGTCGAGGCAGGCC
TTTTCGCGGGCTGGGTTAAGCAGATCGTGAATGACTTCGTCATCGAAGCGAACAAGTC
CAACACCATGGACAAGATCGCGGATATTTCTCTCATCGTACCGTACATCGGCCTGGCG
CTGAATGTTGGCAATGAAACGGCTAAAGGCAACTTCGAAAACGCTTTTGAGATCGCC
GGTGCTTCAATTCTGTTAGAATTTATTCCGGAACTGCTGATCCCGGTGGTTGGCGCATT
TCTGTTGGAGAGCTACATCGACAACAAAAACAAGATCATTAAGACGATCGACAACGC
TCTGACCAAGCGTAATGAAAAGTGGTCGGATATGTATGGCTTGATTGTGGCCCAATGG
CTGAGCACCGTGAACACGCAATTTTATACGATTAAGGAAGGTATGTACAAAGCACTGA
ACTACCAGGCGCAGGCTTTAGAAGAGATTATCAAATACCGCTACAACATTTACAGCGA
AAAGGAGAAGTCCAACATCAACATCGACTTTAACGACATTAACAGCAAGTTGAACGA
GGGCATCAATCAAGCCATTGATAACATTAATAACTTTATCAACGGCTGTAGCGTTAGCT
ACCTGATGAAAAAAATGATCCCGCTGGCAGTTGAGAAGCTGTTGGATTTCGATAACAC
CCTTAAGAAGAATTTACTGAACTACATTGATGAGAACAAGCTGTATTTAATTGGTAGCG
CTGAATATGAAAAATCTAAAGTGAATAAGTATTTAAAGACCATTATGCCTTTTGACCTG
AGCATCTATACCAATGACACCATATTGATTGAGATGTTTAACAAGTACAACAGCGAAAT
CCTGAATAACATAATTCTGAACCTGCGTTACAAAGACAACAATCTCATCGACCTGTCT
GGCTATGGTGCGAAAGTCGAGGTTTACGACGGTGTTGAGCTGAATGACAAGAACCAA
TTTAAGTTAACAAGCAGTGCAAACAGCAAGATCCGCGTGACGCAAAATCAAAATATTA
 
TTTTTAACTCTGTTTTTCTGGATTTCTCTGTTTCCTTCTGGATCCGTATTCCGAAATACA
AAAACGACGGCATTCAGAACTATATCCACAATGAATACACGATCATTAACTGCATGAA
AAATAACAGCGGTTGGAAAATCTCAATCCGTGGCAATCGTATTATCTGGACCCTGATC
GATATCAACGGCAAAACAAAATCCGTGTTCTTTGAATATAACATTCGCGAAGATATCTC
AGAGTATATCAACCGTTGGTTCTTTGTGACGATAACCAATAACTTAAACAACGCGAAA
ATCTATATCAACGGTAAACTTGAGAGCAACACCGACATTAAAGACATCCGCGAAGTTA
TCGCGAACGGTGAGATTATTTTCAAATTGGACGGTGATATCGACCGCACCCAATTCATT
TGGATGAAATATTTCTCCATCTTCAACACCGAGCTGTCCCAATCGAACATTGAGGAAC
GTTATAAGATCCAGAGCTACTCGGAGTACCTGAAAGACTTCTGGGGTAACCCGCTGAT
GTATAACAAAGAGTATTACATGTTTAACGCGGGCAACAAGAACAGCTACATCAAATTG
AAAAAAGATTCCCCGGTTGGTGAAATTCTCACCCGTTCGAAATACAATCAGAATAGCA
AGTATATTAATTATAGAGACCTGTATATTGGCGAGAAGTTTATCATCCGTAGAAAGTCTA
ACAGCCAGAGCATTAATGATGATATCGTTCGTAAAGAGGATTATATCTACTTGGATTTTT
TCAATCTGAACCAAGAATGGCGTGTGTACACGTACAAGTACTTCAAAAAGGAGGAAG
AGAAGTTATTCTTGGCACCGATCAGCGACTCCGACGAGTTCTATAATACCATTCAAATC
AAGGAGTATGACGAGCAGCCAACTTATTCCTGTCAGCTGTTGTTTAAAAAAGATGAAG
AGAGTACCGACGAAATTGGTCTGATCGGCATCCACCGTTTTTATGAGTCCGGCATTGT
GTTCGAAGAGTACAAGGATTACTTTTGCATAAGCAAATGGTATCTGAAAGAGGTGAAG
CGTAAACCGTACAACCTGAAATTGGGCTGCAACTGGCAGTTTATCCCCAAAGATGAG
GGATGGACCGAG
SEQ ID NO: 17 
MPKINSFNYNDPVNDRTILYIKPGGCQEFYKSFNIMKNIWIIPERNVIGTTPQDFH
PPTSLKNGDSSYYDPNYLQSDEEKDRFLKIVTKIFNRINNNLSGGILLEELSKANPYLGND
NTPDNQFHIGDASAVEIKFSNGSQDILLPNVIIMGAEPDLFETNSSNISLRNNYMPSNHRFG
SIAIVTFSPEYSFRFNDNCMNEFIQDPALTLMHELIHSLHGLYGAKGITTKYTITQKQNPLIT
NIRGTNIEEFLTFGGTDLNIITSAQSNDIYTNLLADYKKIASKLSKVQVSNPLLNPYKDVFE
AKYGLDKDASGIYSVNINKFNDIFKKLYSFTEFDLRTKFQVKCRQTYIGQYKYFKLSNLL
NDSIYNISEGYNINNLKVNFRGQNANLNPRIITPITGRGLVKKIIRFCKNIV
SEQ ID NO: 18 
SICIEINNGELFFVASENSYNDDNINTPKEIDDTVTSNNNYENDLDQVILNFNSES
APGLSDEKLNLTIQNDAYIPKYDSNGTSDIEQHDVNELNVFFYLDAQKVPEGENNVNLTS
SIDTALLEQPKIYTFFSSEFINNVNKPVQAALFVSWIQQVLVDFTTEANQKSTVDKIADISI
VVPYIGLALNIGNEAQKGNFKDALELLGAGILLEFEPELLIPTILVFTIKSFLGSSDNKNKVI
KAINNALKERDEKWKEVYSFIVSNWMTKINTQFNKRKEQMYQALQNQVNAIKTIIESKY
NSYTLEEKNELTNKYDIKQIENELNQKVSIAMNNIDRFLTESSISYLMKIINEVKINKLREY
DENVKTYLLNYIIQHGSILGESQQELNSMVTDTLNNSIPFKLSSYTDDKILISYFNKFFKRI
KSSSVLNMRYKNDKYVDTSGYDSNININGDVYKYPTNKNQFGIYNDKLSEVNISQNDYII
YDNKYKNFSISFWVRIPNYDNKIVNVNNEYTIINCMRDNNSGWKVSLNHNEIIWTFEDN
RGINQKLAFNYGNANGISDYINKWIFVTITNDRLGDSKLYINGNLIDQKSILNLGNIHVSD
NILFKIVNCSYTRYIGIRYFNIFDKELDETEIQTLYSNEPNTNILKDFWGNYLLYDKEYYLL
NVLKPNNFIDRRKDSTLSINNIRSTILLANRLYSGIKVKIQRVNNSSTNDNLVRKNDQVYIN
FVASKTHLFPLYADTATTNKEKTIKISSSGNRFNQVVVMNSVGNCTMNFKNNNGNNIGLL
GFKADTVVASTWYYTHMRDHTNSNGCFWNFISEEHGWQEK
SEQ ID NO: 19 
MPKINSFNYNDPVNDRTILYIKPGGCQEFYKSFNIMKNIWIIPERNVIGTTPQDFH
PPTSLKNGDSSYYDPNYLQSDEEKDRFLKIVTKIFNRINNNLSGGILLEELSKANPYLGND
NTPDNQFHIGDASAVEIKFSNGSQDILLPNVIIMGAEPDLFETNSSNISLRNNYMPSNHRFG
SIAIVTFSPEYSFRFNDNCMNEFIQDPALTLMHELIHSLHGLYGAKGITTKYTITQKQNPLIT
NIRGTNIEEFLTFGGTDLNIITSAQSNDIYTNLLADYKKIASKLSKVQVSNPLLNPYKDVFE
AKYGLDKDASGIYSVNINKFNDIFKKLYSFTEFDLRTKFQVKCRQTYIGQYKYFKLSNLL
NDSIYNISEGYNINNLKVNFRGQNANLNPRIITPITGRGLVKKIIRFCKNIVKRGTEEAEKL
GHHHHHHHGTEEAEKLGKRSICIEINNGELFFVASENSYNDDNINTPKEIDDTVTSNNN
YENDLDQVILNFNSESAPGLSDEKLNLTIQNDAYIPKYDSNGTSDIEQHDVNELNVFFYLD
AQKVPEGENNVNLTSSIDTALLEQPKIYTFFSSEFINNVNKPVQAALFVSWIQQVLVDFTT
EANQKSTVDKIADISIVVPYIGLALNIGNEAQKGNFKDALELLGAGILLEFEPELLIPTILVF
TIKSFLGSSDNKNKVIKAINNALKERDEKWKEVYSFIVSNWMTKINTQFNKRKEQMYQA
LQNQVNAIKTIIESKYNSYTLEEKNELTNKYDIKQIENELNQKVSIAMNNIDRFLTESSISY
LMKIINEVKINKLREYDENVKTYLLNYIIQHGSILGESQQELNSMVTDTLNNSIPFKLSSYT
DDKILISYFNKFFKRIKSSSVLNMRYKNDKYVDTSGYDSNININGDVYKYPTNKNQFGIY
 
NDKLSEVNISQNDYIIYDNKYKNFSISFWVRIPNYDNKIVNVNNEYTIINCMRDNNSGWK
VSLNHNEIIWTFEDNRGINQKLAFNYGNANGISDYINKWIFVTITNDRLGDSKLYINGNLI
DQKSILNLGNIHVSDNILFKIVNCSYTRYIGIRYFNIFDKELDETEIQTLYSNEPNTNILKDF
WGNYLLYDKEYYLLNVLKPNNFIDRRKDSTLSINNIRSTILLANRLYSGIKVKIQRVNNSS
TNDNLVRKNDQVYINFVASKTHLFPLYADTATTNKEKTIKISSSGNRFNQVVVMNSVGNC
TMNFKNNNGNNIGLLGFKADTVVASTWYYTHMRDHTNSNGCFWNFISEEHGWQEK
SEQ ID NO: 20

ATGCCCAAAATAAATTCATTTAATTATAACGACCCAGTTAATGACCGCACGAT
CTTGTACATTAAGCCGGGTGGCTGCCAGGAATTCTATAAGTCCTTTAATATAATGAAAA
ACATTTGGATCATCCCGGAACGTAATGTCATTGGCACTACGCCGCAGGACTTCCATCC
GCCAACCAGCCTCAAGAATGGTGACTCTAGCTACTACGACCCGAATTACCTGCAAAGC
GACGAGGAAAAGGACAGATTTCTGAAAATTGTGACCAAAATTTTTAACCGCATTAAC
AACAACCTATCGGGCGGCATCTTGCTTGAAGAATTAAGCAAGGCGAACCCTTATCTGG
GTAACGACAACACCCCAGATAATCAATTTCATATTGGTGACGCGAGCGCTGTGGAAAT
TAAGTTTAGCAATGGCTCTCAGGACATTCTTCTGCCGAACGTCATCATCATGGGTGCA
GAACCGGATCTGTTTGAGACAAACAGCTCCAACATTTCTCTGCGTAACAACTATATGC
CGAGCAACCATCGTTTCGGTAGCATCGCGATTGTGACCTTTTCCCCGGAGTACAGCTT
CCGCTTCAATGACAACTGCATGAACGAGTTCATCCAAGATCCGGCACTGACTCTGATG
CATGAATTAATCCACAGCCTGCATGGTTTATACGGCGCTAAAGGCATTACCACCAAATA
TACCATCACCCAGAAGCAAAACCCACTGATTACGAACATACGTGGTACGAACATCGAG
GAGTTCCTGACGTTCGGCGGTACGGATCTAAATATCATTACCAGTGCGCAAAGCAACG
ACATCTACACGAATTTACTCGCGGATTACAAAAAAATCGCCAGCAAACTTTCGAAGGT
GCAGGTGAGCAATCCGCTGCTGAACCCGTATAAAGATGTTTTTGAGGCGAAATACGGT
CTGGATAAAGACGCCAGCGGTATTTACAGCGTCAATATTAACAAATTCAATGACATCTT
CAAAAAGCTGTACTCCTTCACCGAGTTCGATCTGCGTACCAAGTTCCAGGTTAAGTGC
CGTCAAACCTACATTGGTCAATATAAGTACTTCAAGCTCAGCAATCTGCTGAACGACT
CAATTTATAATATCAGCGAAGGTTATAACATCAACAACCTGAAAGTCAACTTCCGTGGT
CAGAACGCGAACCTAAATCCGCGTATTATTACCCCAATCACCGGCCGTGGTCTGGTTA
AAAAGATTATTCGTTTCTGCAAAAACATTGTTAAAAGAGGCACCGAGGAGGCGGAAA
AATTGGGCCATCACCACCACCACCACCACGGACCGAGGAAGCGGAAAAGCTG

GGAAAACGTAGTATTTGTATTGAAATCAACAACGGCGAACTGTTCTTCGTGGCTAGCG
AAAACAGCTATAACGACGACAATATTAACACTCCGAAAGAGATTGACGACACCGTCA
CCTCAAACAATAACTATGAAAATGACCTGGATCAGGTGATTTTGAACTTTAACAGCGA
ATCTGCACCGGGTCTGTCCGACGAGAAACTGAACCTGACCATCCAAAACGACGCGTA
TATCCCGAAATATGACAGCAATGGTACGTCCGACATCGAACAGCATGATGTGAACGAG
CTGAACGTTTTTTTCTACCTTGATGCCCAAAAGGTGCCGGAGGGTGAAAACAATGTTA
ATCTGACCAGCAGCATCGATACCGCACTGCTGGAGCAGCCGAAGATTTATACTTTCTT
CAGCTCCGAATTCATTAACAACGTGAACAAGCCGGTTCAGGCGGCACTGTTCGTTTCT
TGGATTCAGCAGGTTCTGGTTGACTTTACCACCGAAGCGAATCAGAAGTCTACGGTCG
ATAAAATCGCTGACATCTCTATTGTGGTCCCGTATATCGGCTTGGCCCTTAACATTGGC
AACGAAGCGCAGAAGGGCAACTTCAAGGACGCATTGGAGTTGCTGGGTGCGGGTATC
CTATTAGAGTTCGAGCCGGAGCTGTTGATCCCGACCATCTTGGTTTTTACCATTAAGAG
CTTCCTGGGTAGCTCAGACAACAAGAACAAGGTTATCAAAGCGATTAATAACGCACTG
AAGGAGCGCGATGAGAAATGGAAAGAAGTTTATAGTTTTATCGTGAGCAACTGGATG
ACCAAAATCAACACCCAATTTAATAAACGCAAAGAGCAGATGTATCAGGCTCTGCAG
AACCAGGTAAATGCCATCAAGACGATTATCGAGAGCAAATATAACAGCTACACGCTGG
AGGAAAAGAACGAACTGACCAACAAGTACGACATCAAACAGATTGAGAATGAGCTG
AATCAGAAAGTATCTATCGCTATGAATAACATCGATCGTTTTCTGACGGAAAGCTCGAT
TTCGTACCTTATGAAAATCATCAATGAGGTTAAAATCAATAAGCTTCGTGAATACGATG
AGAACGTCAAAACCTATCTGCTGAATTACATCATTCAGCACGGCTCTATCCTGGGCGA
ATCTCAGCAAGAGCTGAACAGCATGGTTACTGACACCCTGAACAACAGCATCCCGTT
CAAGTTGTCCTCCTACACCGATGACAAAATTTTGATCTCCTATTTCAATAAGTTCTTCA
AGCGCATAAAGTCCAGCTCCGTTTTGAACATGCGCTACAAGAACGACAAGTACGTGG
ACACTAGCGGTTATGATAGCAACATCAATATCAATGGCGATGTCTATAAGTACCCGACC
AACAAGAATCAATTTGGCATTTACAATGACAAATTGTCTGAGGTGAACATTTCTCAAA
ACGACTACATTATCTATGATAATAAGTACAAGAACTTTTCCATTTCGTTCTGGGTACGTA
TTCCCAACTATGATAATAAGATCGTTAACGTTAATAATGAGTACACCATTATCAACTGTA
TGCGCGATAACAACAGCGGTTGGAAAGTTTCTCTGAACCACAACGAAATTATCTGGA
CCTTCGAAGATAATCGTGGCATCAACCAAAAGTTGGCGTTTAACTACGGTAACGCGAA
TGGCATCTCTGATTATATCAATAAATGGATCTTTGTGACCATTACGAACGATCGCCTGG
GCGATTCGAAGTTGTACATCAATGGCAACCTCATCGATCAGAAGAGCATCCTGAACTT

GGGCAACATTCACGTGAGCGACAACATCCTGTTCAAGATCGTGAACTGCAGCTACAC
CCGTTATATCGGTATTCGCTATTTTAACATCTTTGATAAAGAACTGGATGAGACAGAGA
TCCAGACCCTGTATAGCAACGAACCGAATACCAATATCCTGAAGGATTTTTGGGGTAAT
TACTTGCTGTACGACAAAGAATACTACCTGCTGAACGTGTTGAAGCCGAACAACTTCA
TCGACAGACGTAAAGATAGCACCTTGAGTATAAACAATATCCGCTCAACTATCCTGCTG
GCTAACCGTCTGTACTCCGGTATTAAAGTCAAAATTCAGCGTGTTAACAACTCGTCAA
CAAACGACAACCTGGTTCGTAAGAACGACCAAGTGTACATCAATTTTGTTGCTTCCAA
AACCCACCTGTTCCCGCTGTATGCAGATACTGCGACCACCAACAAGGAGAAGACGAT
CAAAATCTCTAGCAGCGGCAACCGTTTCAACCAAGTGGTGGTGATGAACAGCGTGGG
TAATTGCACCATGAATTTCAAGAACAATAATGGTAATAACATCGGTCTGTTGGGCTTTA
AAGCGGATACCGTTGTAGCCAGCACCTGGTATTACACCCATATGCGTGATCATACCAAC
TCTAACGGCTGTTTTTGGAATTTTATTTCGGAAGAGCACGGTTGGCAAGAAAAA

## Claims

1. A recombinant polypeptide, comprising a light chain of a neurotoxin and/or a heavy chain of a neurotoxin, and a linker peptide;
wherein the linker peptide comprises a tag peptide and at least one or two protease cleavage site sequences selected from the group consisting of: a first protease cleavage site sequence and a second protease cleavage site sequence;
wherein, after the first protease cleavage site sequence is recognized by a protease, the protease cleaves the recombinant polypeptide at a junction between the light chain and the linker peptide, and/or after the second protease cleavage site sequence is recognized by a protease, the protease cleaves the recombinant polypeptide at a junction between the linker peptide and the heavy chain.

2. The recombinant polypeptide according to claim 1, wherein the recombinant polypeptide comprises a light chain of a neurotoxin, a heavy chain of a neurotoxin and a linker peptide located between the light chain and the heavy chain, for example, the recombinant polypeptide comprises, from N-terminus to C-terminus, the light chain of the neurotoxin, the linker peptide and the heavy chain of the neurotoxin, wherein the light chain and the heavy chain are linked into a single polypeptide chain by the linker peptide.

3. The recombinant polypeptide according to claim 1 or 2, wherein the recombinant polypeptide comprises a light chain of a neurotoxin linked to a linker peptide, for example, the recombinant polypeptide comprises, from N-terminus to C-terminus, the light chain of the neurotoxin and the linker peptide, and/or
the recombinant polypeptide comprises a heavy chain of a neurotoxin linked to a linker peptide, for example, the recombinant polypeptide comprises, from N-terminus to C-terminus, the linker peptide and the heavy chain of the neurotoxin,
wherein the linker peptide linked to the light chain and the linker peptide linked to the heavy chain are identical or different.

4. The recombinant polypeptide according to claim 1 or 3, wherein the light chain and the heavy chain are located in separate polypeptide chains.

5. The recombinant polypeptide according to any one of claims 1 to 4, wherein the neurotoxin is selected from the group consisting of botulinum neurotoxin, tetanus neurotoxin, and diphtheria toxin.

6. The recombinant polypeptide according to claim 5, wherein the botulinum neurotoxin is selected from the group consisting of botulinum neurotoxin serotypes A, B, C, D, E, F and G, preferably selected from the group consisting of botulinum neurotoxin serotypes A, B and E, for example botulinum neurotoxin serotype A.

7. The recombinant polypeptide according to any one of claims 1 to 6, wherein the light chain has an amino acid sequence as set forth in SEQ ID NO: 1, and/or the heavy chain has an amino acid sequence as set forth in SEQ ID NO: 2.

8. The recombinant polypeptide according to any one of claims 1 to 7, wherein the first protease cleavage site sequence and the second protease cleavage site sequence are each independently selected from the group consisting of cleavage site sequences of Kex2 protease, EK protease and TEV protease;
for example, wherein the first protease cleavage site sequence is selected from the group consisting of R, K, RR, KK, RK and KR, and/or the second protease cleavage site sequence is selected from the group consisting of R, K, RR, KK, RK, KR and DDDDK.

9. The recombinant polypeptide according to claim 8, wherein both the first protease cleavage site sequence and the second protease cleavage site sequence are a Kex2 protease cleavage site sequence.

10. The recombinant polypeptide according to any one of claims 1 to 9, wherein the tag peptide is selected from the group consisting of a polyhistidine tag (poly(His)-tag), a FLAG tag, a streptavidin-binding tag (Strep-tag) and a glutathione S-transferase tag (GST-tag).

11. The recombinant polypeptide according to claim 10, wherein the tag peptide is a polyhistidine tag (poly(His)-tag), for example, a 4, 5, 6, 7, 8, 9 or 10×His-tag.

12. The recombinant polypeptide according to any one of claims 1 to 11, wherein the recombinant polypeptide further comprises a first auxiliary sequence, wherein the first auxiliary sequence is located between the first protease cleavage site sequence and the tag peptide.

13. The recombinant polypeptide according to any one of claims 1 to 12, wherein the recombinant polypeptide further comprises a second auxiliary sequence, wherein the second auxiliary sequence is located between the second protease cleavage site sequence and the tag peptide.

14. The recombinant polypeptide according to claim 12 or 13, wherein the first auxiliary sequence and the second auxiliary sequence are each independently in a length of at least 1, 2, 3, 4, 5, 6, 7, 8, 9 or 10 amino acids.

15. The recombinant polypeptide according to any one of claims 12 to 14, wherein the first auxiliary sequence and the second auxiliary sequence are each independently selected from the group consisting of GSGS, EEGSGS, GSGSDDGSGS, GSGSEDGSGS, GSGSDEGSGS, EEAE, DDAD, TEEAEKL, TDDADKL, TEDAEKL, TDEADKL, GTEEAEKLG, EGTEEAEKLG, EGTDDADKLG, EGTEEADKLG, EGTDDAEKLG, EGTEDADKLG and EGTDEAEKLG, and sequences having at least 60%, 70%, 80%, or 90% identity thereto.

16. An isolated polynucleotide, encoding the recombinant polypeptide according to any one of claims 1 to 15.

17. An expression vector, comprising the polynucleotide according to claim 16.

18. A cell, comprising the polynucleotide according to claim 16 or the expression vector according to claim 17.

19. The cell according to claim 18, wherein the cell is selected from the group consisting of a prokaryotic cell and a eukaryotic cell; for example, the prokaryotic cell is selected from *Escherichia coli* cells; for example, the eukaryotic cell is selected from the group consisting of yeast cells, insect cells, plant cells and mammalian cells.

20. A method for producing a neurotoxin, comprising culturing the cell according to claim 18 or 19 under conditions suitable for expression of the recombinant polypeptide.

21. The method according to claim 20, wherein the method further comprises isolating the recombinant polypeptide expressed recombinantly, and cleaving the recombinant polypeptide with a protease.

22. The method according to claim 20 or 21, wherein the method further comprises purifying the neurotoxin from cell culture.

23. A pharmaceutical or cosmetic composition, comprising the neurotoxin produced by the method according to any one of claims 20 to 22.

24. Use of the recombinant polypeptide according to any one of claims 1 to 15, the polynucleotide according to claim 16, the expression vector according to claim 17, or the cell according to claim 18 or 19 in the manufacture of a neurotoxin, for example, the neurotoxin is selected from the group consisting of tetanus neurotoxin, diphtheria toxin, and botulinum neurotoxin.
